# EUROPEAN PATENT APPLICATION

(11) **EP 1 245 236 A1**
(43) Date of publication of application: **02.10.2002**
(21) Application number: 00985979.4
(22) Date of filing: 28.12.2000
(51) Int. Cl.: A61K 38/17, A61K 38/40, A61K 38/21, A61P 1/16, A61P 31/20, C07K 19/00, C07K 14/705, C07K 14/79, C07K 14/555

(54) **PREVENTIVES AND REMEDIES FOR CHRONIC HEPATITIS**

(30) Priority: 28.12.1999 JP 37408799
(71) Applicant: Sumitomo Pharmaceuticals Company, Limited, Osaka-shi, Osaka 541-8510 (JP)
(72) Inventor: TOHDOH, Naoki, Higashinada-ku Kobe-shi Hyogo 658-0056 (JP); MURATA, Masashi, Osaka 561-0859 (JP); ENJOJI, Takashi, Osaka 569-0857 (JP)
(74) Representative: Cresswell, Thomas Anthony
(86) International application number: JP0009393
(87) International publication number: WO01047545

(57) **Abstract**

To provide a prophylactic or therapeutic agent for chronic hepatitis, capable of inhibiting the binding of hepatitis virus and a target cell, thereby preventing or treating chronic hepatitis; and a fusion interferon capable of still more improving a viral clearance ratio at the termination of interferon administration. A prophylactic or therapeutic agent for chronic hepatitis, comprising an oligopeptide having the following characteristics: (A) having a binding affinity to a viral antigen protein; (B) inhibiting binding of virus to a receptor protein on a target cell for said virus; and (C) having homologies with said receptor protein at the amino acid sequence level; a fusion interferon containing an amino acid sequence of the above-mentioned oligopeptide; and method for treating chronic hepatitis in combination with the use of interferon, characterized by using the oligopeptide or a part thereof in combination with interferon.

## Description

### TECHNICAL FIELD

The present invention relates to a therapeutic agent for chronic hepatitis, comprising an oligonucleotide haying an amino acid sequence or a part thereof, wherein the amino acid sequence is important for infection of hepatitis virus into a target cell. Concretely, the present invention relates to a therapeutic agent for chronic hepatitis, comprising an oligopeptide or a part thereof, the oligopeptide being capable of binding to an antigenic protein for hepatitis virus and having an action of inhibiting binding of virus to a receptor protein, or a compound prepared on the basis of its sequence. Also, the present invention relates to a fusion interferon comprising the oligopeptide and its partial amino acid sequence. Further, the present invention relates to a method for treating chronic hepatitis in combination with the use of interferon.

### BACKGROUND ART

Chronic type-B hepatitis and type-C hepatitis developed by infection with hepatitis B virus (HBV) and hepatitis C virus (HCV) are grave viral diseases which are transformed into cirrhosis or hepatocellular carcinoma at a high ratio. In Japan, the number of hepatitis B virus carriers is said to be about one million, and that of hepatic C virus carriers is said to be about 1.3 millions. The carriers have been spread world-widely. HCV, which is a single-stranded RNA virus belonging to Flaviviridae, has been considered to transform to chronicity by factors for the virus side such as avoidance from the immune mechanism by gene mutation and factors from the host side such as decrease in the immunocompetence and the viral clearance ability. On the other hand, since HBV belonging to Hepadonaviridae is a double-stranded DNA virus, a mutation in a virus genome does not take place as much as it does in HCV; however, it transforms to chronicity by incorporation into chromosome DNA. Also in HBV, there is found fulmination caused by avoidance from the immune mechanism caused by mutation of a virus gene.

Presently, the only one treatment method for eliminating the virus is an interferon treatment method. However, a ratio at which the virus is finally eliminated is 40% or so in HCV and 20% or so in HBV. In particular, in an interferon treatment method for the HCV, there is caused a difference in its therapeutic effect depending upon the status of the patient, such as serotype of HCV, virus amount and histological progression degree. In particular, in the case of genotype II and hyperviremia, it has been known that its amelioration effect is low and a virus clearance ratio is also low.

As a method of treating chronic type-C hepatitis, a treatment mainly comprising clearance of hepatitis C virus (HCV) and improvement in liver function is carried out. Inter alia, a treatment of chronic type-C hepatitis by interferon having improved actions in both HCV clearance and liver function is carried out as the only one method of treatment for virus clearance. However, such a treatment has the disadvantage that a virus complete clearance ratio for HCV is as low as 40% or so. For that reason, a method for treating chronic hepatitis in combination with the use of interferon and ribavirin, amantadine or the like has been recently developed; however, the method still has a disadvantage that the effectiveness is yet low. Alternatively, a drug for improving a liver function by using glycyrrhizin or the like has been tried for a patient showing resistance to interferon; however, the drug has a disadvantage that the liver function is rapidly worsened after the termination of administration.

On the other hand, as a method of treating chronic type-B hepatitis, an interferon treatment method is carried out as in the case of chronic type-C hepatitis; however, the therapeutic effect can be expected currently on the level of only 20% or so. Recently, in the treatment of chronic type-B hepatitis, an inhibitor for an HBV polymerase has been administered; however, the administration has the large side effects and relapse of virus is found after the termination of administration. Further, upon the development of fulminant type-B hepatitis, a method for treatment in combination with the use of interferon and cyclosporin is carried out after exchange of whole serum; however, a lifesaving rate is low currently. As described above, a treatment method which is truly effective for chronic type-B hepatitis and chronic type-C hepatitis which may lead to development of cirrhosis or hepatocellular carcinoma has not been established currently, even though hepatitis B virus and hepatitis C virus have been distributed world-widely and virus carriers are present at 1% or so or greater of the population.

Accordingly, the present invention has been accomplished in view of the above-mentioned conventional problems, and a first object of the present invention is to provide a prophylactic or therapeutic agent for chronic hepatitis, capable of inhibiting the binding of hepatitis virus and a target cell, thereby preventing or treating chronic hepatitis. A second object of the present invention is to provide a fusion interferon and a method for treating chronic hepatitis in combination with the use of interferon, capable of still more improving a viral clearance ratio at the termination of interferon administration.

### DISCLOSURE OF INVENTION

Concretely, the gist of the present invention relates to:
[1] a prophylactic or therapeutic agent for chronic hepatitis, comprising an oligopeptide having the following characteristics:
   (A) having a binding affinity to a viral antigen protein;
   (B) inhibiting binding of virus to a receptor protein on a target cell for the virus; and
   (C) having homologies with the receptor protein at the amino acid sequence level;
[2] a prophylactic or therapeutic agent for chronic type-C hepatitis, comprising a compound having the following characteristics:
   (a) having a binding affinity to an envelope protein of HCV; and
   (b) inhibiting binding of HCV to a receptor protein on a target cell for the HCV;
[3] a prophylactic or therapeutic agent for chronic type-B hepatitis, comprising a compound having the following characteristics:
   (a') having a binding affinity to S antigen protein of HBV; and
   (b') inhibiting binding of HBV to a receptor protein on a target cell for the HBV;
[4] a fusion interferon comprising an amino acid sequence of an oligopeptide having the following characteristics:
   (A) having a binding affinity to a viral antigen protein;
   (B) inhibiting binding of virus to a receptor protein on a target cell for the virus; and
   (C) having homologies with the receptor protein at the amino acid sequence level; and
[5] a method for treating chronic hepatitis in combination with the use of interferon, characterized by using an oligopeptide or a part thereof in combination with interferon, wherein the oligopeptide has the following characteristics:
   (A) having a binding affinity to a viral antigen protein;
   (B) inhibiting binding of virus to a receptor protein on a target cell for the virus; and
   (C) having homologies with the receptor protein at the amino acid sequence level.

### BRIEF DESCRIPTION OF THE DRAWINGS

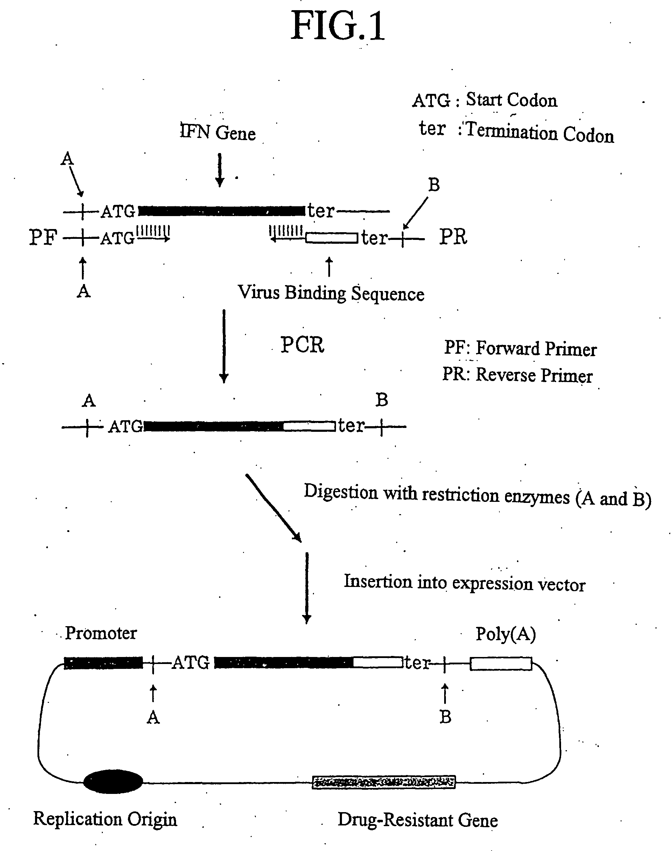

Figure 1 shows a schematic view of a construct of a gene for interferon with a corresponding oligopeptide added on a C-terminal side.

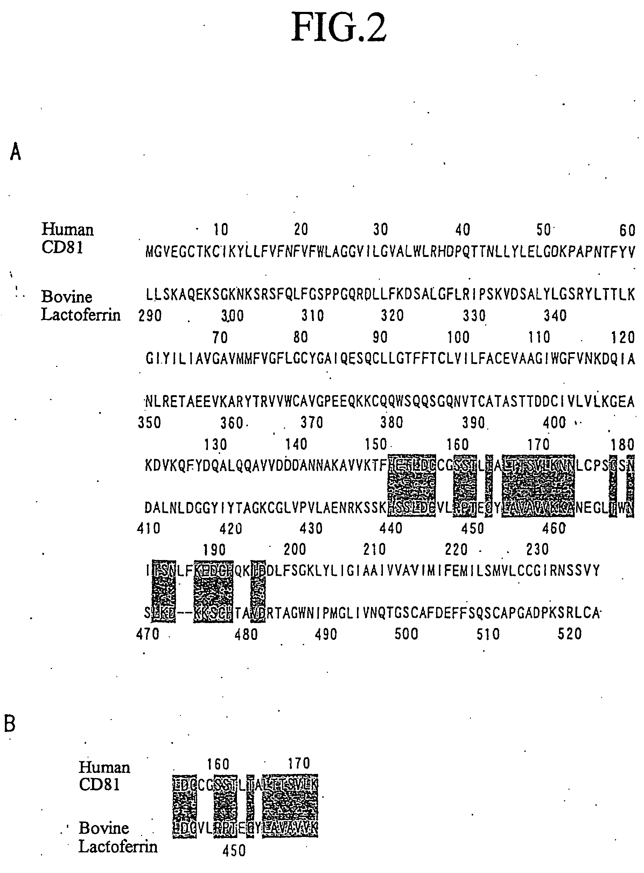

Figure 2 is a diagram showing the analytical results on the homologies between an amino acid sequence of human CD81 and that of bovine lactoferrin. In Panel A, an upper row shows an amino acid sequence of human-derived CD81, and a lower row an amino acid sequence of bovine lactoferrin, and a homologous region between human CD81 and bovine lactoferrin is shaded with meshed pattern. Panel B shows a region which is mostly conserved in the homologous region shown by Panel A.

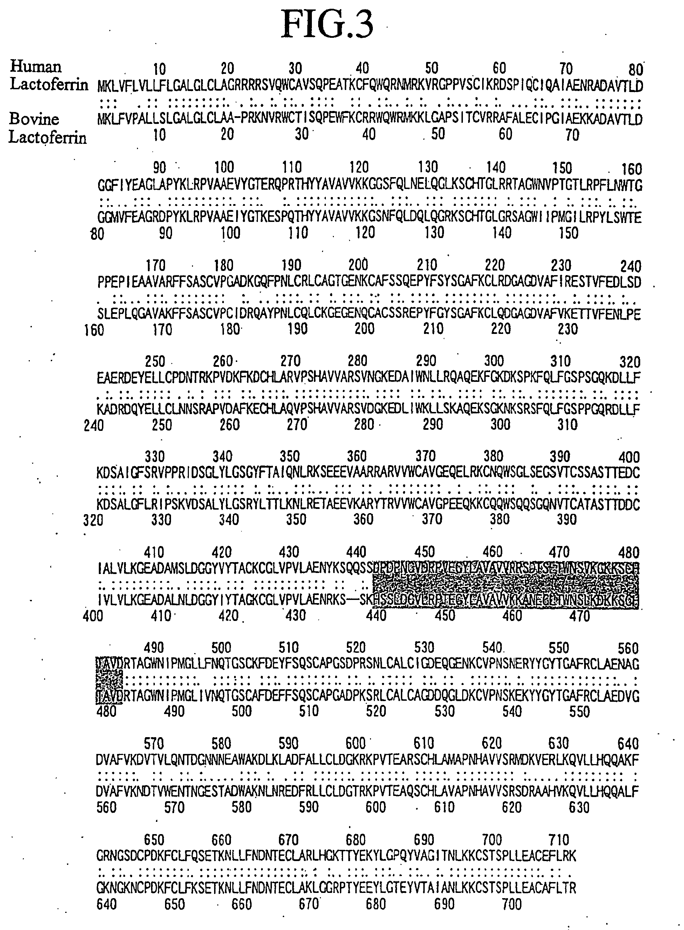

Figure 3 is a diagram showing the analytical results on the homologies between an amino acid sequence of bovine lactoferrin and that of human lactoferrin. An upper row shows bovine lactoferrin and a lower row shows human lactoferrin. In the figure, a region showing the homologies with CD81 is shown.

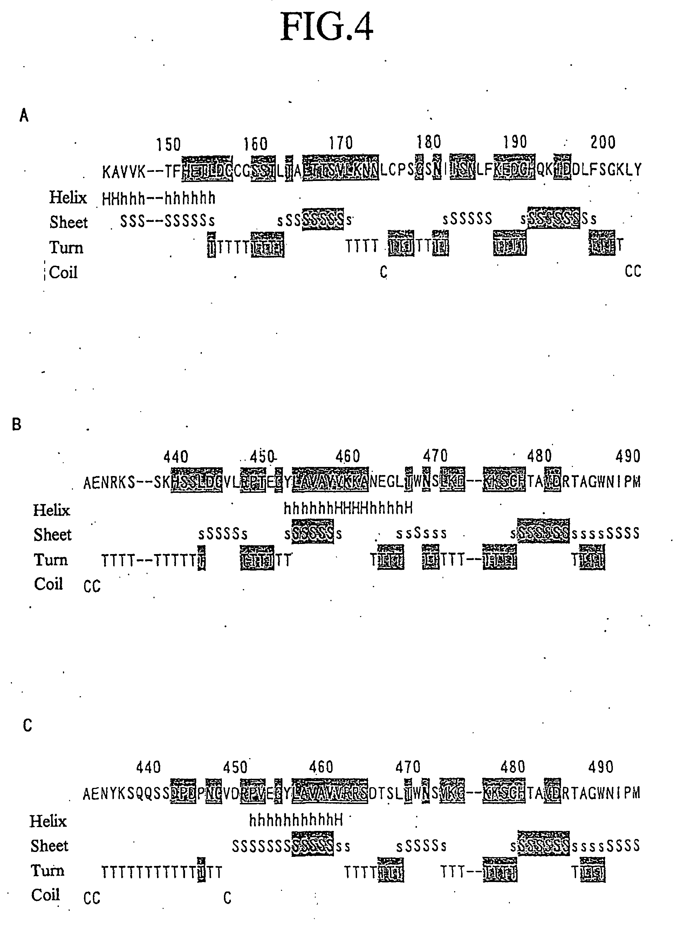

Figure 4 shows a diagram predicting secondary structure of a partial amino acid sequence of a homologous sequence between human CD81 and bovine lactoferrin, which are considered to be bound to HCV. In the figure, among the homologous sequences, the identical amino acids are indicated by bold characters, and the homologous amino acids are indicated by plain characters. In addition, in the prediction of the secondary structure, a part corresponding to amino acids constituting the same secondary structure between human CD81 and bovine lactoferrin is shaded with meshed pattern. In the case of human lactoferrin, a sequence homologous to human CD81 and the identical portions of amino acids maintaining the common secondary structure between human CD81 and human lactoferrin in the amino acid sequence are shaded with meshed pattern. Panel A shows a binding site of human CD81, Panel B shows a binding site of bovine lactoferrin, and Panel C shows a binding site of human lactoferrin.

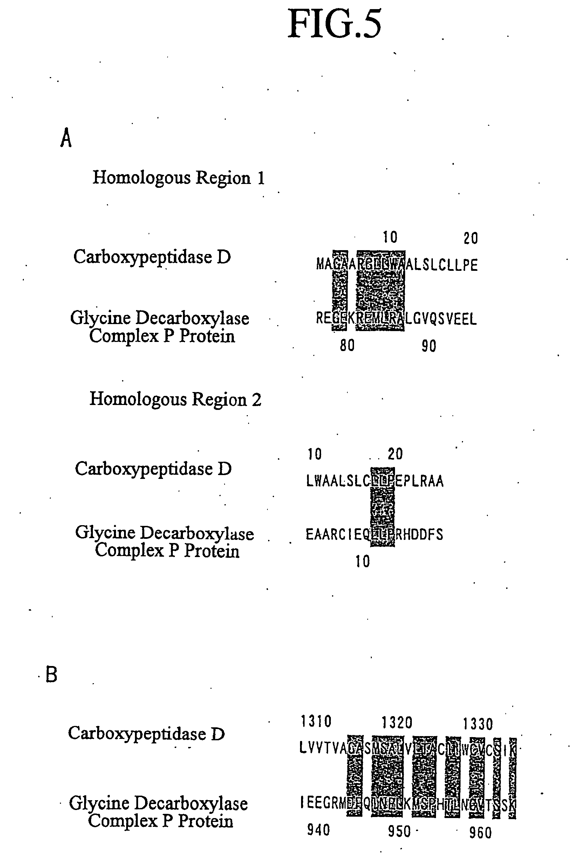

Figure 5 is a diagram showing the analytical results on the homologies of an amino acid sequence of a binding region, on the basis of an amino acid sequence of duck-derived carboxypeptidase D and duck-derived glycine decarboxylase complex P protein, in which binding to duck HBV pre-S antigen is suggested. Panel A shows the analytical results on N-terminal side, and Panel B shows the analytical results on C-terminal side.

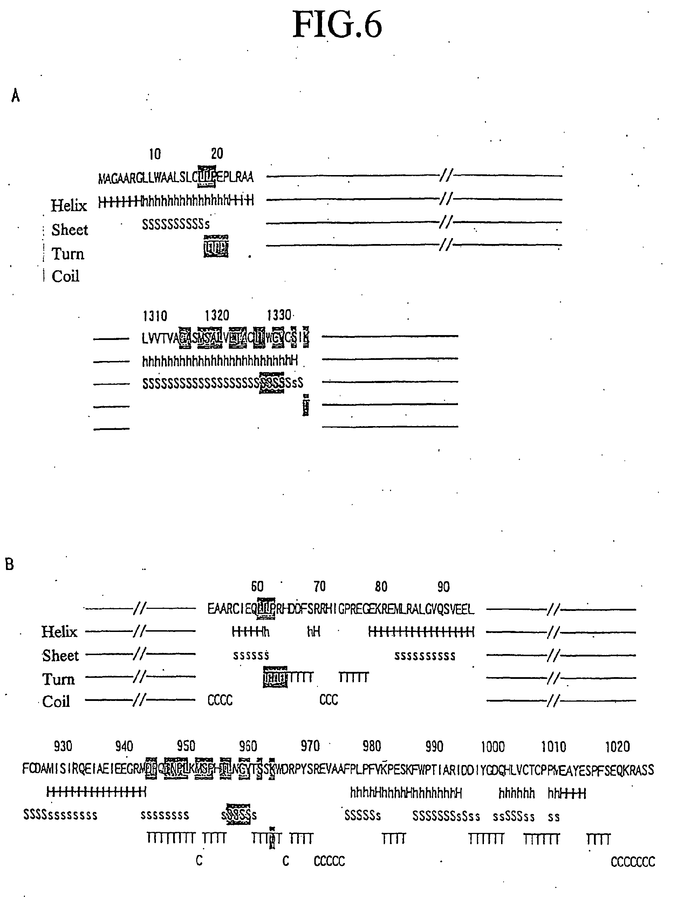

Figure 6 shows a diagram predicting a secondary structure of the homologous region in Figure 5. Panel A shows the analytical results on N-terminal side, and Panel B shows the analytical results on C-terminal side.

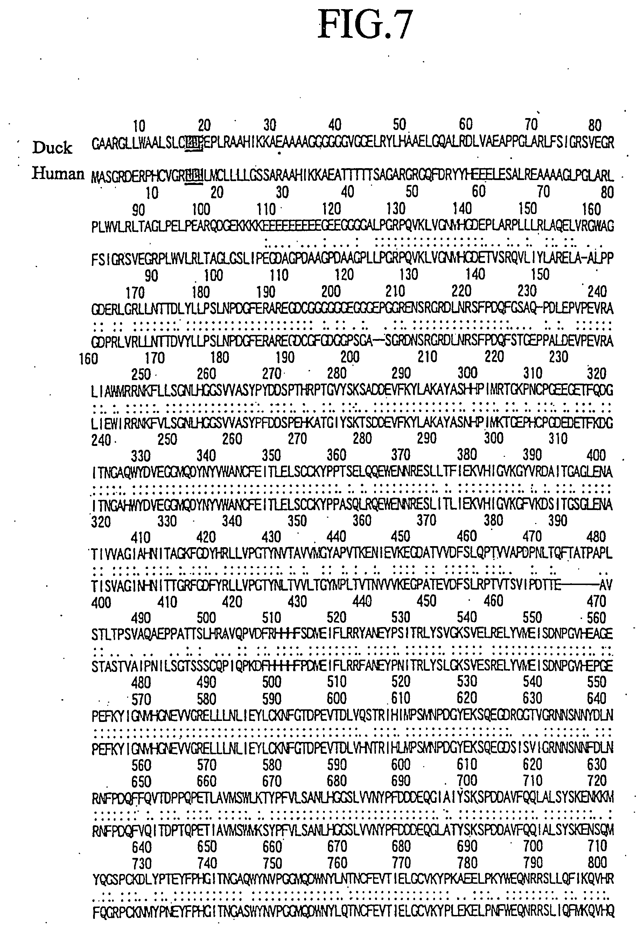

Figure 7 shows a diagram showing the analytical results on the homologies between an amino acid sequence of a binding region of duck-derived carboxypeptidase D to duck HBV and an amino acid sequence of human-derived carboxypeptidase D.

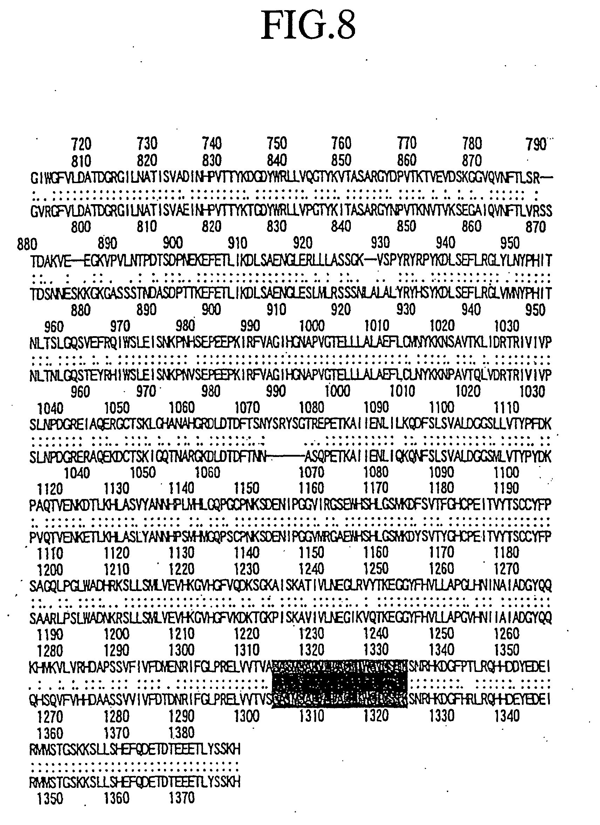

Figure 8 shows a diagram showing the analytical results on the homologies between an amino acid sequence of a binding region of duck-derived carboxypeptidase D to duck HBV and amino acid sequence of human-derived carboxypeptidase D. Figure 8 is a continuation from the analytical results shown in Figure 7.

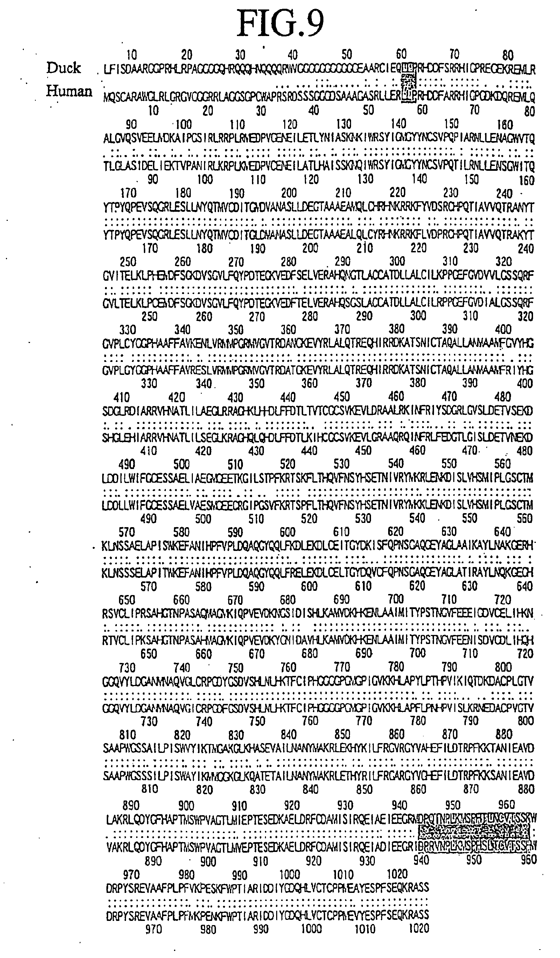

Figure 9 shows the analytical results on the homologies between an amino acid sequence of duck-derived glycine decarboxylase complex P protein and an amino acid sequence of human-derived glycine decarboxylase complex P protein.

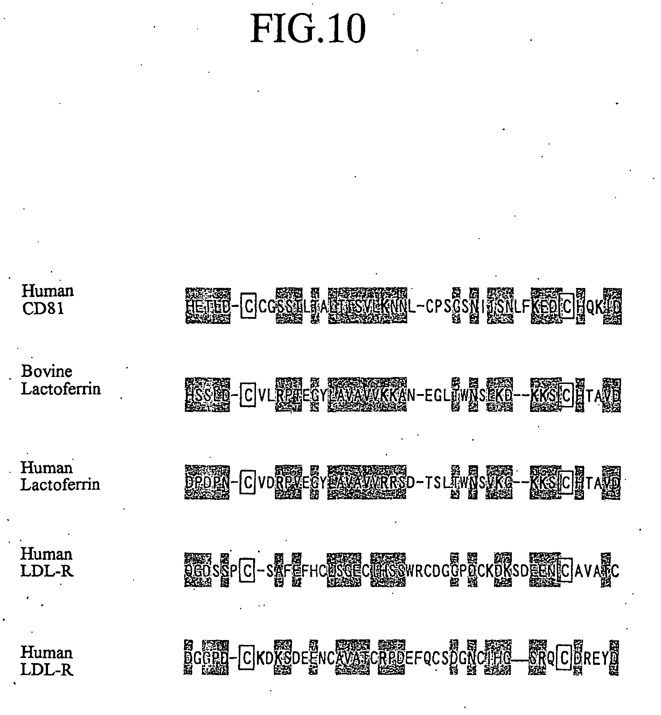

Figure 10 is a diagram showing the analytical results on the homologous regions of human CD81, bovine lactoferrin, human lactoferrin and human LDL receptor. C circumscribed by an open square means a cysteine residue which is commonly found in all the sequences. Basic amino acids contained in respective sequences are shown by bold characters. Amino acid residues which are homologous in three of the sequence of human CD81 (SEQ ID NO: 1), the sequence of bovine lactoferrin (SEQ ID NO: 2) and the sequence of human lactoferrin (SEQ ID NO: 3) are shaded with meshed pattern. In the sequence of human LDL receptor (SEQ ID NO: 18) in an upper row, amino acid residues which are homologous to those of human CD81 are shaded with meshed pattern. In the sequence of human LDL receptor (SEQ ID NO: 19) in a lower row, residues which are homologous to those of human lactoferrin are shaded with meshed pattern.

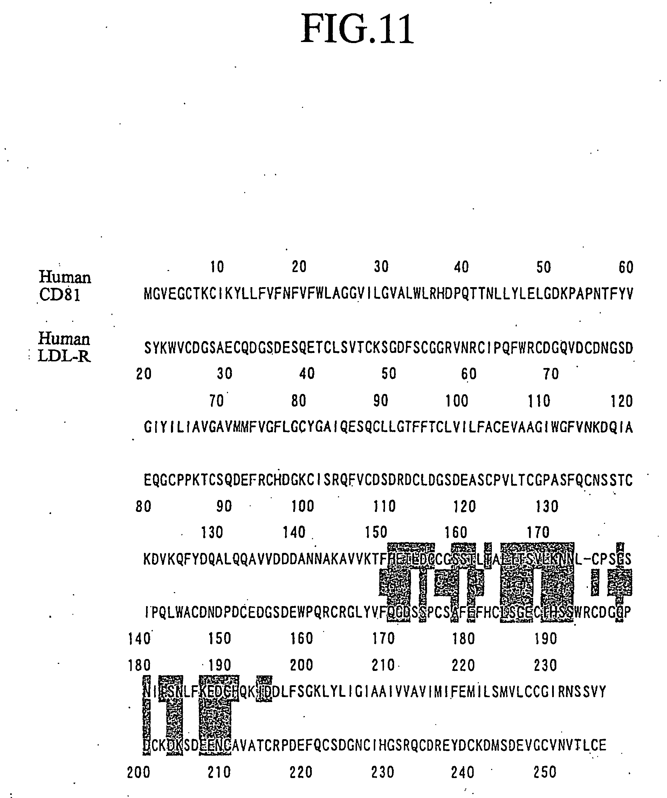

Figure 11 is a diagram showing the analytical results on a homologous region between an amino acid sequence of human CD81 and that of human LDL receptor.

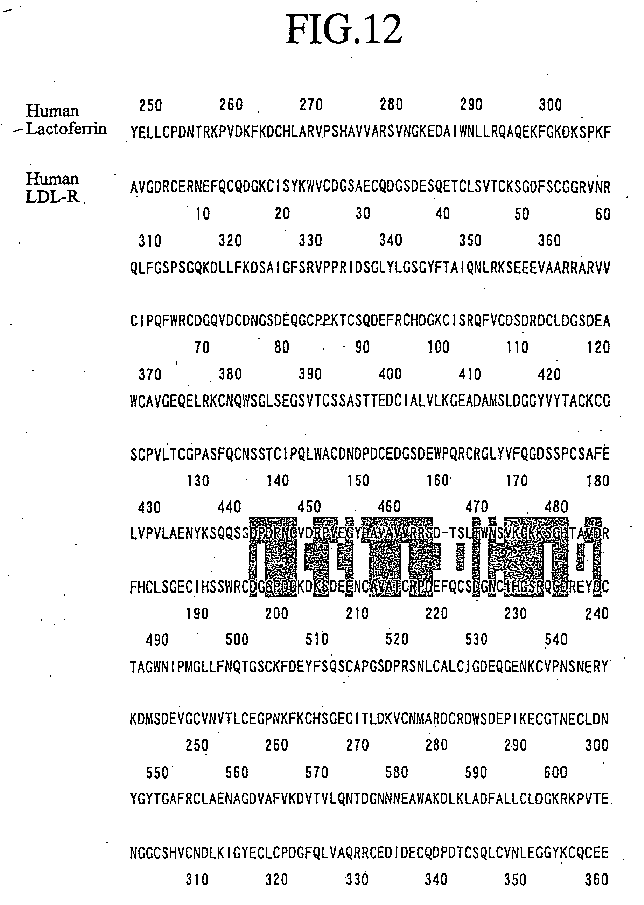

Figure 12 is a diagram showing the analytical results on a homologous region between an amino acid sequence of human lactoferrin and that of human LDL receptor.

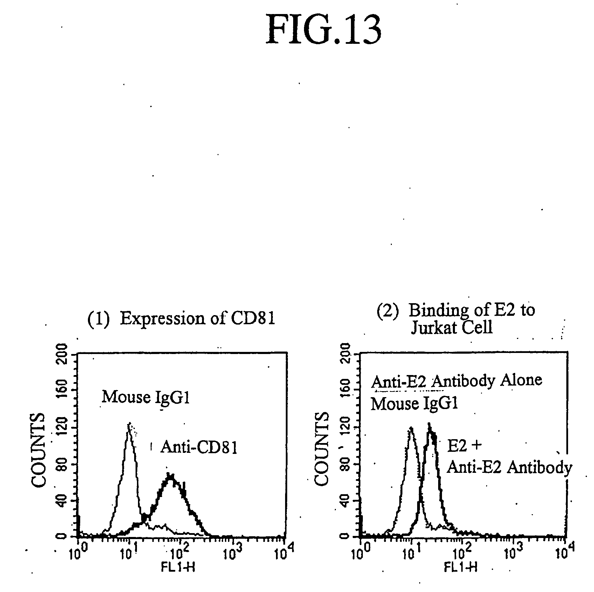

Figure 13 is diagrams showing the results of CD81 expression analyzed by FACScan. Panel (1) is a histogram obtained by staining Jurkat cells with an anti-human CD81 antibody and an FITC-labeled anti-mouse IgG1 antibody, and analyzing by FACScan. A horizontal axis represents FITC fluorescence intensity and a vertical axis represents the number of cells. The results of staining with an isotype control antibody (mouse IgG1), which is a negative control in staining, is shown by a fine solid line, and the results of staining with an anti-human CD81 antibody is shown by a bold solid line. On the other hand, Panel (2) is a histogram obtained by analyzing binding of the recombinant E2 protein to the Jurkat cells by FACScan. The results of staining with the mouse IgG1 is shown by a fine solid line; the results obtained by staining the Jurkat cells untreated with the recombinant E2 protein, with an anti-E2 antibody is shown by a dotted line; and the results obtained by staining the Jurkat cells treated with the recombinant E2 protein, with an anti-E2 antibody is shown by a bold solid line.

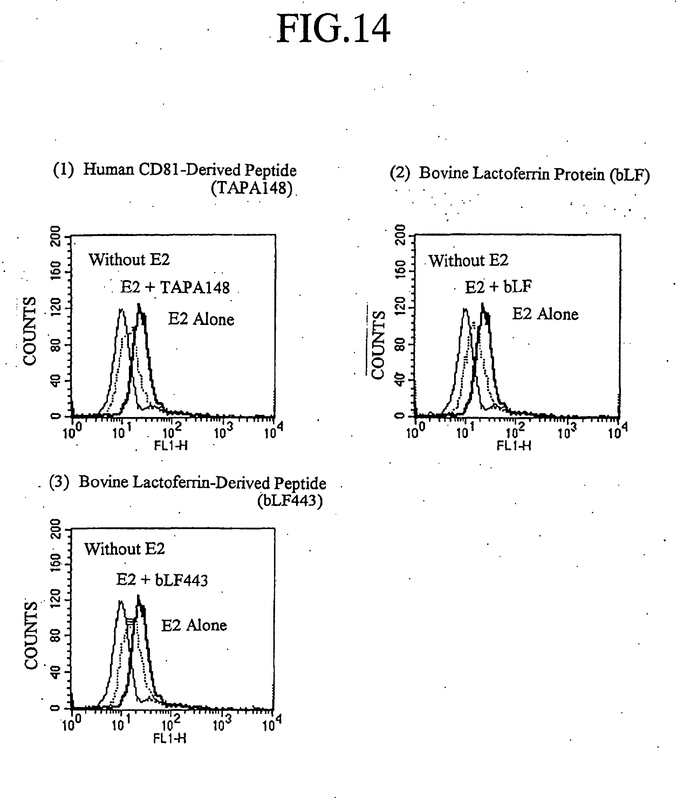

Figure 14 is a histogram obtained by analyzing with FACScan the binding to the Jurkat cells of the recombinant E2 protein previously incubated with the human CD81-derived peptide [Panel (1)], the bovine lactoferrin protein [Panel (2)] or the bovine lactoferrin-derived peptide [Panel (3)]. The results for which the Jurkat cells without treatment of the recombinant E2 protein were stained with an anti-E2 antibody is shown by a thin solid line, the results for which the Jurkat cells treated with the recombinant E2 protein alone were stained with the anti-E2 antibody is shown by a bold solid line, and the results for which the Jurkat cells treated with the recombinant E2 protein preincubated with a peptide or a protein were stained with the anti-E2 antibody is shown by a dotted line.

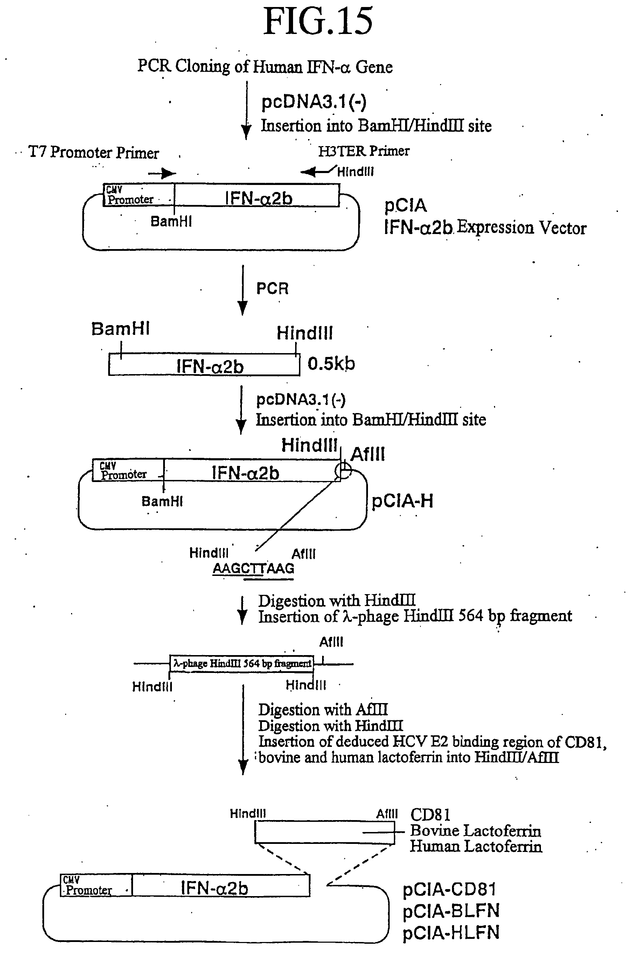

Figure 15 is a diagram showing the procedures for constructing respective expression vectors for IFN-α2b, IFN-CD81, IFN-human lactoferrin and IFN-bovine lactoferrin, which are HCV binding type IFNs.

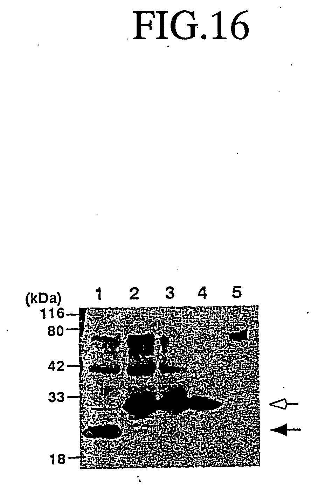

Figure 16 is a schematic diagram showing the results of Western blot analysis with an anti-IFN-α antibody, regarding IFN-α2b, and IFN-CD81, IFN-human lactoferrin and IFN-bovine lactoferrin, which are HCV binding type IFNs, wherein each of them is obtained by protein synthesis with *in vitro* transcription/translation by using the prepared expression vector as a template. Lane 1: pCIA, lane 2: pCIA-CD81, lane 3: pCIA-HLFN, lane 4: pCIA-BLFN, and lane 5: Luciferase T7 control DNA. A solid black arrow shows IFN-α2b, and an open arrow shows the HCV binding type IFNs.

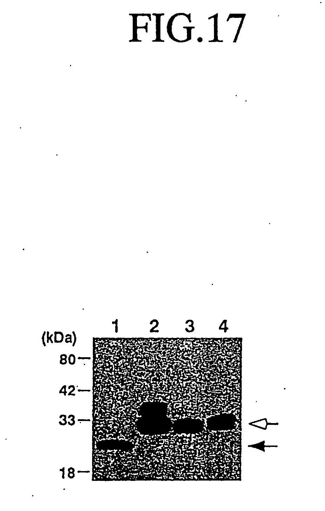

Figure 17 is a schematic diagram showing the results of Western blot analysis with an anti-IFN-α antibody, regarding IFN-α2b, and IFN-CD81, IFN-human lactoferrin and IFN-bovine lactoferrin, which are HCV binding type IFNs, wherein each of them is obtained by transient expression in COS-1 cells. Lane 1: pCIA, lane 2: pCIA-CD81, lane 3: pCIA-HLFN, and lane 4: pCIA-BLFN. A solid black arrow shows IFN-α2b, and an open arrow shows the HCV binding type IFNs.

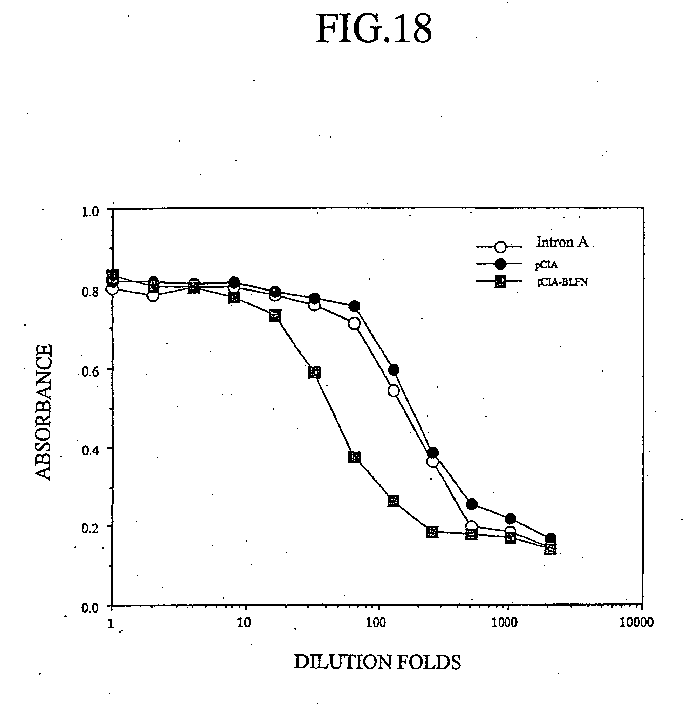

Figure 18 is a diagram showing the anti-viral activity of the culture supernatant of COS-1 cells three days after transfection of pCIA and pCIA-BLFN. In the system for evaluating the Sindbis virus-FL cells, the evaluation was carried out by a Crystal Violet method. INTRON A was 2-fold stepwise diluted where 200 IU/ml was regarded as a dilution fold 1. The culture supernatants were 2-fold stepwise diluted where 1000-fold dilution with the medium was regarded as a dilution fold 1. A vertical axis shows the absorbance of the cells stained with Crystal Violet at 595 nm.

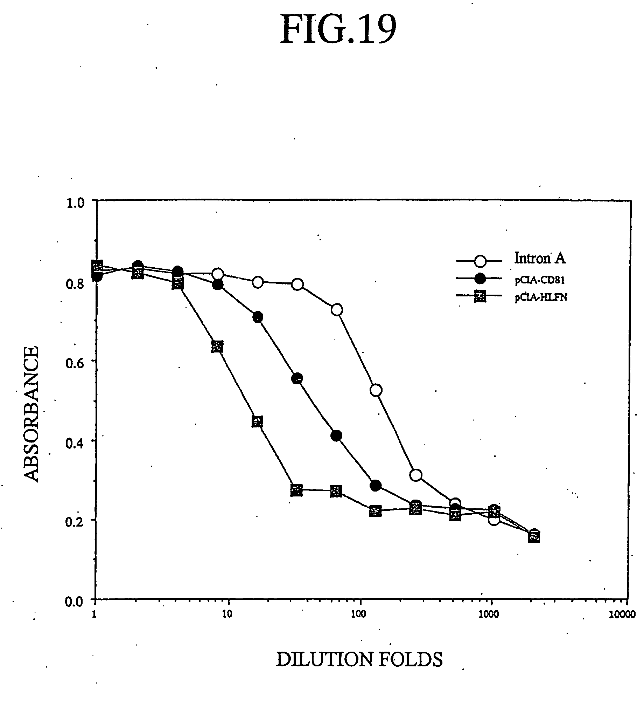

Figure 19 is a diagram showing the anti-viral activity of the culture supernatant of the COS-1 cells three days after transfecion of pCIA-CD81 and pCIA-HLFN. In the system for evaluating the Sindbis virus-FL cells, the evaluation was carried out using a Crystal Violet method. INTRON A was 2-fold stepwise diluted where 200 IU/ml was regarded as a dilution fold 1. The culture supernatants were 2-fold stepwise diluted where 1000-fold dilution with the medium was regarded as a dilution fold 1. A vertical axis shows the absorbance of the cells stained with Crystal Violet at 595 nm.

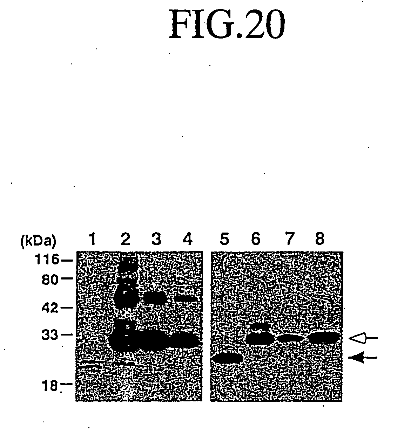

Figure 20 is a schematic diagram showing the binding of transiently expressed HCV binding type IFNs in COS-1 cells to baculovirus-derived recombinant GST-fusion HCV E2 protein (GST-E2). Each of GST-E2 and IFN-α2b (lanes 1 and 5), IFN-CD81 (lanes 2 and 6), IFN-human lactoferrin (lanes 3 and 7), and IFN-bovine lactoferrin (lanes 4 and 8), which are HCV-binding type IFNs, was incubated, and co-precipitated with Glutathione beads, and thereafter the detection was carried out using an anti-IFN-α antibody. Lanes 1 to 4 are precipitation fractions, and lanes 5 to 8 are supernatant fractions. A solid black arrow shows IFN-α2b, and an open arrow shows HCV-binding type IFNs.

### MODES FOR CARRYING OUT THE INVENTION

One of the features of the prophylactic or therapeutic agent for chronic hepatitis of the present invention resides in that the prophylactic or therapeutic agent comprises an oligopeptide having the following characteristics:
(A) having a binding affinity for a viral antigen protein;
(B) inhibiting binding of virus to a receptor protein on a target cell for the virus; and
(C) having homologies with the receptor protein at the amino acid sequence level. Since the prophylactic or therapeutic agent for chronic hepatitis of the present invention comprises the oligopeptide having the above-mentioned characteristics (A) to (C), it is considered that the binding of hepatitis virus, especially hepatitis C virus or hepatitis B virus, to a target cell is inhibited. Therefore, there are exhibited excellent effects that chronic hepatitis can be prevented or treated.

In the present specification, hepatitis C virus will be abbreviated as "HCV" and hepatitis B virus will be abbreviated as "HBV."

The viral antigen protein includes, for instance, an envelope protein of HCV, pre-S protein of HBV, and the like. In addition, the concept of the present invention can be applied to diseases other than chronic hepatitis. In this case, the envelope protein of HIV or the like can be used as a viral antigen protein.

Since the oligopeptide having the above-mentioned characteristics (A) to (C) comprises an amino acid sequence essential for binding of a receptor protein to virus, the binding affinity of a viral antigen protein to an oligopeptide is exhibited. It is desired that the above-mentioned binding affinity is higher than the binding affinity of virus to a receptor protein on a target cell for the virus, from the viewpoint of inhibiting the binding of the virus to the receptor protein on the target cell for the virus.

The receptor protein of a target cell for the virus includes human CD81, which is a receptor molecule for HCV; carboxypeptidase D, to which pre-S protein of HBV is deduced to be bound; glycine decarboxylase complex P protein and the like. In the present invention, human CD81, human carboxypeptidase D and glycine decarboxylase complex P protein are preferable.

At the amino acid sequence level, the above-mentioned oligopeptide has the homologies with a receptor protein of 20% or more at a homologous amino acid level, from the viewpoint of exhibiting sufficient binding affinity. In addition, in the case where similar amino acids are included, it is preferable that the homologies are 50% or more.

The present invention has been accomplished on the basis of the technical idea described below.

With respect to HCV, which is an etiologist for hepatitis C, it has been reported that some antibodies to a hypervariable region (HVR1) which is a part of E2 region of an envelope protein of HCV weakens the infectivity of virus (Rosa D. et al., *Proc. Natl. Acad. Sci. USA*., 1996, **93**, 1759-1763). A receptor of the cell for HCV is CD81, and it has been suggested that the binding is mediated by E2 region of HCV (Pileri P. et al., *Science,* 1998, **282**, 938-941). In addition, it has been shown that the binding to a viral receptor is inhibited not only by an antibody against HVR1, but other region is necessary for the binding. In addition, aggregation of cells is observed due to binding of an antibody against E2 region or CD81 (Flint M. et al., *J. Virol.*, 1999, **73**, 6235-6244).

In addition, recently, it has been reported that lactoferrin contained in breast milk binds to HCV and reduces an amount of virus in blood (Tanaka K. et al., *Jpn. J. Cancer Res.,* 1999, **90**, 367-371). It has been further reported that a viral region to which lactoferrin binds is E2 region (Akito Nozaki et al., *Society of Japan Virology, the* *47th academic meeting, general meeting program, abstract,* 1999, 188). Further, it has reported that the LDL receptor has an affinity with E2 region [Wunschmann, S. et al., *J. Virol*., **74**,10055-10062 (2000)].

Since a half-life of virus in blood is deduced to be 2.7 hours (Neumann AU. et al., *Science,* 1998, **282**, 103-107), virus in blood rapidly disappears, when the infection to a cell is suppressed. Standing on this point of view, it is considered that suppression of adhesion of virus to a target cell is effective for eliminating virus in blood in a shorter period of time. However, unless the infected cell is eliminated as described above, complete disappearance of virus cannot be accomplished. Immediately after the administration of interferon, virus in blood is rapidly eliminated, but release of a small amount of virus produces a new infection. Therefore, it is considered that virus is relapsed after the completion of administration of interferon. Therefore, it is considered that a clearance of virus by interferon enhanced by additional administration of a substance which inhibits the infection of virus itself to the administration of interferon having both an anti-virus action and an immuno-stimulating action.

In addition, in the case of HBV, which is an etiologist for hepatitis B, since HBV is a double-stranded DNA virus, it can be hardly considered that a viral genome disappears directly by an anti-virus action of interferon. Also, since HBV is not a cytotoxic virus and an amount of the virus in blood is correlated with a degree of liver dysfunction, it is considered that the liver dysfunction due to HBV is mainly caused by the immunological eliminating action for a viral infected cell. It is considered that an interferon treatment finally eliminates the virus by mainly more effectively enhancing this immunological eliminating action.

HBV is a relatively stable virus. It is considered that if the infection to a target cell can be inhibited, a new infection is protected, whereby the immunological eliminating action in an interferon treatment can be maximally drawn out.

Recently, host proteins binding to pre-S protein of duck hepatitis B virus have been identified. The host proteins were carboxypeptidase D and glycine decarboxylase complex P protein (Tong S. et al., *J. Virol.*, 1999, **73**, 8696-8702 and Li J. et al., *J. Biol. Chem.,* 1999, **274**, 27658-27665). There has not yet been verified the presence or absence of the affinity of these proteins with pre-S protein in human.

The present inventors have assumed that there is a sequence capable of inhibiting the binding of CD81 as a HCV receptor to HCV in an amino acid sequence of lactoferrin, and thought that the sequence has certain homologies with CD81. In addition, in HBV, the affinity of HBV pre-S to a cell is present in a limited region from 83rd amino acid residue to 107th amino acid residue in the pre-S protein (Tong S. et al., *J. Virol.*, 1999, **73**, 8696-8702 and Li J. et al., *J. Biol. Chem.*, 1999, **274**, 27658-27665). Therefore, even if there are a plurality of proteins having an affinity to pre-S proteins, the present inventors have deduced that their binding reactions are the same, and thought that there are found some homologies in an amino acid sequence and a tertiary structure in carboxypeptidase D and glycine decarboxylase complex P protein. As a result, the present inventors have found a partially similar sequence from the comparison of an amino acid sequence of lactoferrin, which is thought to protect against the HCV infection, with an amino acid sequence of human CD81, which is a viral receptor, and also found an amino acid sequence which is similar to that of a protein having an affinity to duck HBV pre-S. The present inventors have thought that an oligopeptide sequence or a part thereof, or a compound having a structure similar to tertiary structures of those sequences has been effective for suppressing the viral infection. The present invention has been found based on such a discussion.

The prophylactic or therapeutic agent for chronic hepatitis of the present invention include a prophylactic or therapeutic agent for chronic type-C hepatitis, a prophylactic or therapeutic agent for chronic type-B hepatitis, and the like.

One of the features of the prophylactic or therapeutic agent for chronic type-C hepatitis of the present invention resides in that the prophylactic or therapeutic agent comprises a compound having the following characteristics:
(a) having a binding affinity to an envelop protein of HCV; and
(b) inhibiting binding of HCV to a receptor protein on a target cell for the HCV. Since the prophylactic or therapeutic agent for chronic type-C hepatitis of the present invention comprises a compound having the above-mentioned characteristics (a) and (b), it is considered that the binding of hepatitis C virus on a target cell is inhibited. Therefore, there are exhibited excellent effects that chronic type-C hepatitis can be prevented or treated.

Concretely, there can be exemplified as the compound having the above-mentioned characteristics (a) and (b), oligopeptides (1) and (2) described below, nucleic acids encoding the oligopeptides, and the like.

(1) Oligopeptide comprising an amino acid sequence or a part thereof, wherein the amino acid sequence is found by identification of a homologous sequence of bovine and human lactoferrins which has been known to bind to HCV, and human CD81, which is a receptor molecule for HCV.

Concretely, there are exemplified oligopeptides shown in the following 1) to 3), which are a region for binding to HCV, the oligopeptides being capable of inhibiting the binding to a viral receptor:
1) an oligopeptide having a part of human-derived CD81 protein (SEQ ID NO: 7), wherein the oligopeptide consists of the amino acid sequence of SEQ ID NO: 1;
2) an oligopeptide having a part of bovine-derived lactoferrin protein
   (SEQ ID NO: 8), wherein the oligopeptide consists of the amino acid sequence of SEQ ID NO: 2; and
3) an oligopeptide having a part of human-derived lactoferrin protein
   (SEQ ID NO: 9), wherein the oligopeptide consists of the amino acid sequence of SEQ ID NO: 3.

The nucleic acid encoding an oligopeptide satisfying the above-mentioned requirement 1) includes a nucleic acid comprising a nucleic acid selected from the group consisting of:
(i) a nucleic acid encoding an oligopeptide having the amino acid sequence of SEQ ID NO: 1;
(ii) a nucleic acid having the nucleotide sequence of SEQ ID NO: 4; and
(iii) a part of the nucleic acid encoding an oligopeptide having the amino acid sequence of SEQ ID NO: 1.

In addition, the nucleic acid encoding an oligopeptide satisfying the above-mentioned requirement 2) includes a nucleic acid comprising a nucleic acid selected from the group consisting of:
(iv) a nucleic acid encoding an oligopeptide having the amino acid sequence of SEQ ID NO: 2;
(v) a nucleic acid having the nucleotide sequence of SEQ ID NO: 5; and
(vi) a part of a nucleic acid encoding an oligopeptide consisting of the amino acid sequence of SEQ ID NO: 2.

In addition, the nucleic acid encoding an oligopeptide satisfying the above-mentioned requirement 3) includes a nucleic acid comprising a nucleic acid selected from the group consisting of:
(vii) a nucleic acid encoding an oligopeptide having the amino acid sequence of SEQ ID NO: 3;
(viii) a nucleic acid having the nucleotide sequence of SEQ ID NO: 6; and
(ix) a part of the nucleic acid encoding a polypeptide having the amino acid sequence of SEQ ID NO: 3.

(2) Oligopeptide comprising an amino acid sequence or a part thereof, wherein the amino acid sequence is found by identification of a homologous sequence between a human LDL receptor, which has been known to bind to HCV, and human CD81, which is a receptor molecule for HCV.

Concretely, there are exemplified oligopeptides shown in the following 1') and 2'), which are a region for binding to HCV, the oligopeptides being capable of inhibiting binding to a viral receptor:
1') an oligopeptide having a part of a human LDL receptor (SEQ ID NO: 22), wherein the oligopeptide consists of the amino acid sequence of SEQ ID NO: 18; and
2') an oligopeptide having a part of a human LDL receptor (SEQ ID NO: 22), wherein the oligopeptide consists of the amino acid sequence of SEQ ID NO: 19.

The nucleic acid encoding an oligopeptide satisfying the above-mentioned requirement 1') includes a nucleic acid comprising a nucleic acid selected from the group consisting of:
(i') a nucleic acid encoding an oligopeptide having the amino acid sequence of SEQ ID NO: 18;
(ii') a nucleic acid having the nucleotide sequence of SEQ ID NO: 20; and
(iii') a part of the nucleic acid encoding an oligopeptide having the amino acid sequence of SEQ ID NO: 18.

In addition, the nucleic acid encoding an oligopeptide satisfying the above-mentioned requirement 2') includes a nucleic acid comprising a nucleic acid selected from the group consisting of:
(iv') a nucleic acid encoding an oligopeptide having the amino acid sequence of SEQ ID NO: 19;
(v') a nucleic acid having the nucleotide sequence of SEQ ID NO: 21; and
(vi') a part of a nucleic acid encoding an oligopeptide consisting of the amino acid sequence of SEQ ID NO: 19.

The nucleic acid used in the present invention may be a nucleic acid complementary to the nucleic acids of the above-mentioned (i) to (ix) and (i') to (vi'), and concretely the nucleic acid may be DNA or RNA. In addition, as long as the encoded polypeptides have the above-mentioned characteristics (a) and (b), there can also be used in the present invention (x) a nucleic acid having a nucleotide sequence having substitution, deletion, addition or insertion in at least one base in a nucleotide sequence of a nucleic acid selected from the group consisting of the above-mentioned (i) to (ix); (xi) a nucleic acid capable of hybridizing with a nucleic acid selected from the group consisting of above-mentioned (i) to (x) under the stringent conditions; (vii') a nucleic acid having a nucleotide sequence having substitution, deletion, addition or insertion in at least one base in a nucleotide sequence of a nucleic acid selected from the group consisting of the above-mentioned (i') to (vi'); and (viii') a nucleic acid capable of hybridizing with a nucleic acid selected from the group consisting of the above-mentioned (i') to (vi') under the stringent conditions.

The term "stringent conditions" as referred to herein includes conditions described in Sambrook et al., *Molecular Cloning: A Laboratory Manual Second eds*., (1980).

Here, each of the nucleotide sequences of SEQ ID NOs: 7 to 9 is a nucleotide sequence of a nucleic acid encoding human-derived protein CD81, a nucleotide sequence of a nucleic acid encoding bovine-derived lactoferrin or a nucleotide sequence of a nucleic acid encoding human-derived lactoferrin. The amino acid sequence of SEQ ID NO: 1 is a part of an amino acid sequence of human-derived CD81 of SEQ ID NO: 7. Also, the amino acid sequence of SEQ ID NO: 2 is a part of an amino acid sequence of bovine-derived lactoferrin of SEQ ID NO: 8. In addition, the amino acid sequence of SEQ ID NO: 3 is a part of an amino acid sequence of human-derived lactoferrin of SEQ ID NO: 9. The nucleotide sequence of SEQ ID NO: 4 is a nucleotide sequence of a nucleic acid encoding an amino acid sequence of human-derived CD81 of SEQ ID NO: 1; the nucleotide sequence of SEQ ID NO: 5 is a nucleotide sequence of a nucleic acid encoding an amino acid sequence of bovine-derived lactoferrin of SEQ ID NO: 2; and the nucleotide sequence of SEQ ID NO: 6 is a nucleotide sequence of a nucleic acid encoding an amino acid of human-derived lactoferrin of SEQ ID NO: 3. Each of SEQ ID NOs: 18 and 19 is a part of an amino acid sequence of human LDL receptor of SEQ ID NO: 22. Each of SEQ ID NOs: 20 and 21 is a nucleotide sequence of a nucleic acid encoding an amino acid sequence of human-derived LDL receptor of SEQ ID NO: 18 or 19.

Further, the compound which can be used in the prophylactic or therapeutic agent for chronic type-C hepatitis of the present invention includes an oligopeptide or a part thereof, wherein the oligopeptide has the following characteristics:
I) consisting of 31 to 33 amino acids which are flanked by cysteine residues;
II) forming a loop structure; and
III) comprising basic amino acid residues at a middle portion and C-terminal side of loop structure;
a cyclic oligopeptide comprising the oligopeptide or a part thereof; and the like.

Concretely, the oligopeptide having the above-mentioned characteristics I) to III) includes a peptide having the amino acid sequence of SEQ ID NO: 1, 2, 3 or 27, and a cyclic peptide having the amino acid sequence of SEQ ID NO: 28.

The oligopeptide used in the present invention can be deduced by comparison with each of an amino acid sequence of bovine lactoferrin, of which binding activity to HCV is known, and an amino acid sequence of CD81.

Concretely, an amino acid sequence is deduced on the basis of each nucleotide sequence of human CD81 (accession No. NM004356) or bovine lactoferrin (accession No. M63502) disclosed in a known database (GenBank), and each amino acid sequence may be compared.

Each peptide is synthesized on the basis of the resulting homologous region, and binding activity of the peptide to a recombinant E2 protein is confirmed. A confirmation of the binding activity can be carried out, for example, by ELISA, immunoprecipitation, RIA or the like.

Thereafter, inhibition of binding of the recombinant E2 protein to a cell expressing CD81, for example, Jurkat cell may be confirmed. The confirmation of inhibition of the binding can be carried out by FACS analysis, or determination of radioactivity bound to a cell in a case where the recombinant E2 protein labeled with a radioactive isotope element is used, besides the above-mentioned ELISA and RIA.

In addition, in order to determine what sort of an amino acid sequence contained in the oligopeptide is effective for suppressing the virus infection, a partial amino acid sequence of the above-mentioned selected sequence is synthesized, whereby selection can be made on the basis of the binding affinity to E2 and the inhibitory effect of binding of E2 to CD81.

One of the features of the prophylactic or therapeutic agent for chronic type-B hepatitis of the present invention resides in that the prophylactic or therapeutic agent comprises a compound having the following characteristics:
(a') having a binding affinity to S antigen protein of HBV; and
(b') inhibiting binding of HBV to a receptor protein on a target cell for the HBV. Since the prophylactic or therapeutic agent for chronic type-B hepatitis of the present invention comprises the compound having the above-mentioned characteristics (a') and
(b'), it is considered that binding of a hepatitis B virus on a target cell is inhibited. Therefore, there are exhibited excellent effects that chronic type-B hepatitis can be prevented or treated.

Concretely, as the compound having the above-mentioned characteristics (a') and (b'), there are exemplified an oligopeptide described in item (2) below and a nucleic acid encoding the oligopeptide.

(2) Oligopeptide comprising an amino acid sequence or a part thereof, wherein the amino acid sequence is found by identifying a homologous sequence between human carboxypeptidase D and human glycine decarboxylase complex P protein, which are known to bind to HBV

Concretely, there are exemplified oligopeptides shown in the following 1) to 2), which are a region for binding with HBV, the oligopeptides being capable of inhibiting binding to a viral receptor:
1) an oligopeptide having a part of human carboxypeptidase D (SEQ ID NO: 16), wherein the oligopeptide consists of the amino acid sequence of SEQ ID NO: 10; and
2) an oligopeptide having a part of human glycine decarboxylase complex P protein (SEQ ID NO: 17), wherein the oligopeptide consists of the amino acid sequence of SEQ ID NO: 11.

The nucleic acid encoding an oligopeptide satisfying the above-mentioned requirement 1) includes a nucleic acid comprising a nucleic acid selected from the group consisting of:
(I) a nucleic acid encoding an oligopeptide having the amino acid sequence of SEQ ID NO: 10;
(II) a nucleic acid having the nucleotide sequence of SEQ ID NO: 12; and
(III) a part of the nucleic acid encoding an oligopeptide having the amino acid sequence of SEQ ID NO: 10.

The nucleic acid encoding an oligopeptide satisfying the above-mentioned requirement 2) includes a nucleic acid comprising a nucleic acid selected form the group consisting of:
(IV) a nucleic acid encoding an oligopeptide having the amino acid sequence of SEQ ID NO: 11;
(V) a nucleic acid having a nucleotide sequence of SEQ ID NO: 13; and
(VI) a part of the nucleic acid encoding an oligopeptide having the amino acid sequence of SEQ ID NO: 11.

The nucleic acid used in the present invention may be a nucleic acid complementary to the above-mentioned nucleic acids (I) to (VI), and concretely the nucleic acid may be DNA or RNA. In addition, as long as an encoded polypeptide has the above-mentioned characteristics (a') and (b'), there can be also used in the present invention (VII) a nucleic acid having a nucleotide sequence having substitution, deletion, addition or insertion in at least one base in a nucleotide sequence of a nucleic acid selected from the group consisting of the above-mentioned (I) to (VI), and (VIII) a nucleic acid capable of hybridizing with a nucleic acid selected from the group consisting of the above-mentioned (I) to (VII) under the stringent conditions.

Here, each of the amino acid sequences of SEQ ID NOs: 16 and 17 is an amino acid of human-derived carboxypeptidase D and an amino acid of human-derived glycine decarboxylase complex P protein, respectively. The amino acid sequence of SEQ ID NO: 10 is a part of an amino acid sequence of human-derived carboxypeptidase D of SEQ ID NO: 16. In addition, the amino acid sequence of SEQ ID NO: 11 is a nucleotide sequence of a nucleic acid encoding an amino acid sequence of human-derived glycine decarboxylase complex P protein of SEQ ID NO: 17. The nucleotide sequence of SEQ ID NO: 12 is a nucleotide sequence of a DNA encoding an amino acid sequence of human-derived carboxypeptidase D of SEQ ID NO: 10, and the nucleotide sequence of SEQ ID NO: 13 is a nucleotide sequence of a DNA encoding an amino acid sequence of human-derived glycine decarboxylase complex P protein of SEQ ID NO: 11.

The oligopeptide used in the present invention can be deduced, for example, by comparison with each of amino acid sequences of duck-derived carboxypeptidase D, which is considered to be important for binding to duck HBV, and glycine decarboxylase complex P protein.

Concretely, an amino acid sequence is deduced by nucleotide sequences of duck-derived carboxypeptidase D (accession No. AF039749) and glycine decarboxylase complex P protein (accession No. AF137264) disclosed in a known database (GenBank), and each region associated with binding to duck-derived pre-S may be compared. As to an application to human, a duck-derived sequence is compared with an amino acid sequence deduced from nucleotide sequences of human-derived carboxypeptidase D (accession No. U65090) and glycine decarboxylase complex P protein (accession No. NM000170), and an amino acid sequence of a corresponding human-derived peptide may then be determined.

Each peptide is synthesized on the basis of the obtained homologous region, and the binding affinity of the peptide to the recombinant pre-S protein may be confirmed.

An amino acid sequence of a region effective for suppressing the virus infection in an amino acid sequence contained in the oligopeptide used in the prophylactic or therapeutic agent for chronic hepatitis of the present invention, concretely a prophylactic or therapeutic agent for chronic type-C hepatitis or a prophylactic or therapeutic agent for chronic type-B hepatitis, can be selected by synthesizing a peptide consisting of a part of the amino acid sequence selected as mentioned above and selecting on the bases of the binding activity to a viral antigen and the inhibitory effect of binding of a viral antigen to a virus receptor.

The oligopeptide having the selected amino acid sequence itself may be administered to a patient with chronic type-B hepatitis or chronic type-C hepatitis. Further, the oligopeptide having the above-mentioned selected amino acid sequence is thought to have an enhanced virus clearance ratio by using the oligopeptide in combination with interferon.

In the case of chronic type-C hepatitis, soluble CD81 or lactoferrin itself can be administered. It is preferable that an oligopeptide having only a sequence important for binding to HCV is administered, from the viewpoints of reducing the degradation of a protein absorbed during an oral administration and the side effects during intravenous injection.

The same can be said for the case of chronic type-B hepatitis. The soluble carboxypeptidase D or glycine decarboxylase complex P protein itself can be administered. It is preferable that an oligopeptide having only a sequence important for binding to HBV is administered.

A prophylactic or therapeutic agent comprising as an active ingredient the selected oligopeptide or a part of the oligopeptide, or a nucleic acid encoding thereof can be administered in combination with an adjuvant, or can be administered in a particulate preparation. As a dosage form, more concretely, a prophylactic or therapeutic agent can be prepared into the form of a liposome preparation, a particulate preparation binding to a bead having a diameter of a few µm, a preparation binding to a lipid or the like. The dose can be appropriately adjusted depending upon the properties of an infected cell, age and weight of a patient and the like, and is usually 0.001 mg/kg/dosage to 1000 mg/kg/dosage. It is preferable that the prophylactic or therapeutic agent is administered every day at an initial administration, and thereafter administered once in several days to several months depending upon the amount of virus in a cell. As an administration form, the prophylactic or therapeutic agent can be administered by oral administration, intraarterial injection, intravenous injection, intramuscular injection or local injection to the liver.

In addition, it is preferable that the above-mentioned oligopeptide is stabilized in blood, and the oligopeptide may be modified for that purpose. For example, the oligopeptide can be modified by preparing an oligopeptide stable in blood by connecting the oligopeptide to polyethylene glycol, to lengthen its half-life in blood, or modifying the oligopeptide at N-terminal or substituting with D-amino acid.

According to the above-mentioned oligopeptide, there can be further provided a method for treating chronic hepatitis in combination with the use of interferon. The method for treating chronic hepatitis in combination with the use of interferon is also encompassed within the scope of the present invention. Concretely, one of the characteristics of the above-mentioned method for treatment in combination with the use of interferon resides in that the method uses an oligopeptide or a part thereof in combination with interferon, wherein the oligopeptide having the following characteristics:
(A) having a binding affinity to a viral antigen protein;
(B) inhibiting binding of virus to a receptor protein on a target cell for the virus; and
(C) having homologies with the receptor protein at the amino acid sequence level.

The action of interferon is roughly classified into the anti-virus action directly acting on an infected cell and the immuno-stimulating action of host immunity.

For example, in the case of HCV, the anti-virus action by interferon is generated by signal transmission via an interferon receptor, thereby promoting degradation or translation inhibition of RNA of HCV by inducing production of an anti-virus factor. Usually, in an interferon treatment to chronic type-C hepatitis, since RNA of HCV in serum becomes outside the detection limit two days after the administration at 73.8% of remarkably effective cases, it is considered that HCV is eliminated rapidly after the interferon administration (Saito H. et al., *Keio J. Med*., 1997, **46,** 74-80). However, among these cases in which viruses are disappeared, relapse of virus is found after the termination of the interferon administration in a considerable number of cases. These results mean that even if viral RNA is not detected in blood during the interferon administration, many viruses are present in liver or peripheral blood mononuclear cell (PBMC). In the treatment of chronic type-C hepatitis resistant to the interferon treatment, it is thought that complete elimination of HCV present in an infected cell or immunological elimination of an infected cell is necessary. In order to increase the ratio of virus elimination by interferon, activation of these two actions are to be considered. According to the method for treatment in combination with the use of interferon of the present invention, binding of HCV on a target cell can be inhibited by using the above-mentioned oligopeptide or nucleic acid, so that HCV can be eliminated by interferon, whereby the virus clearance ability can be made more effective after the start of the interferon administration. As a result, relapse of interferon-resistant virus at the termination of the interferon administration can be further reduced.

In the present invention, the selected oligopeptide can be fused with another functional molecule to be used as a fusion substance. The fusion substance is also encompassed in the present invention.

In this case, it is preferable that a site into which an oligopeptide sequence is inserted is a position at which the activity of the functional molecule is not reduced. In particular, fusion of an interferon molecule and a selected oligopeptide sequence is advantageous in the interferon treatment in that a single dose administration can be made.

Concretely, the fusion interferon includes a fusion interferon comprising an amino acid sequence of an oligopeptide having the following characteristics:
(A) having a binding affinity to a viral antigen protein;
(B) inhibiting binding of virus to a receptor protein on a target cell for the virus; and
(C) having homologies with the receptor protein at the amino acid sequence level. More specifically, there are included a fusion interferon comprising at least one kind of an amino acid sequence or a part thereof, wherein the amino acid sequence is selected from the group consisting of SEQ ID NOs: 1, 2 and 3; a fusion interferon comprising an amino acid sequence of SEQ ID NO: 10 or 11 or a part thereof.

When an oligopeptide sequence is inserted into an interferon molecule, the corresponding oligopeptide may be conveniently connected to N-terminal or C-terminal of interferon. Since a neighbor region of N-terminal or C-terminal of interferon is not a region essential for binding of interferon to the interferon receptor, it is determined that interferon retains the activity with a high possibility by addition of the oligopeptide sequence to the terminal. However, a structure of a connected amino acid sequence influences the interferon activity in some cases. In that case, an oligopeptide sequence which does not lower the activity of interferon and can bind to a virus antigen may be limited.

In this case, an interferon gene may be amplified using PCR primers comprising a region having homologies with a 5'-terminal side or a 3'-terminal side of a DNA encoding the corresponding oligopeptide and a DNA encoding interferon. In addition, it is desired that to add a restriction enzyme cleavage site to a 5'-side of a primer for insertion into an expression vector, because insertion into an expression vector is conveniently carried out. An example of the construction of an interferon gene with the corresponding oligopeptide added on C-terminal side is shown in Figure 1. Hereinafter, the interferon with the corresponding oligopeptide added on C-terminal is referred to as "C-terminal added interferon," and the interferon with the corresponding oligopeptide added on N-terminal side is referred to as "N-terminal added interferon."

In the production of C-terminal added and N-terminal added interferons, any cell strains derived from prokaryote or eukaryote can be used as a host. In the case of C-terminal added interferon, a termination codon is incorporated at 3'-terminal of a DNA encoding an oligopeptide to be added on C-terminal side. On the other hand, in the case of N-terminal added interferon, when the prokaryote is used as a host, a primer may be designed so that a DNA sequence encoding an oligopeptide having an initiation codon at 5'-terminal is contained. On the other hand, when a cell strain derived from eukaryote is used as a host, a primer may be designed so that an amino acid sequence of an oligopeptide is inserted between C-terminal of a signal sequence of interferon and N-terminal of processed interferon.

The original interferon gene used as a template in reaction for PCR may be any of interferon-α, interferon-β and interferon-γ genes. When an interferon-α gene having many subtypes is used, a gene can be cloned by RT-PCR method from Namalwa cell and the like which have been stimulated and infected with NDV or Sendai virus in accordance with the nucleotide sequences described in the database.

The usefulness of C-terminal added and N-terminal added interferons is as follows:
1) by binding of C-terminal added or N-terminal added interferon to an envelope protein of HCV or S antigen of HBV, the adhesion of HCV or HBV to a cell can be inhibited; therefore, the anti-virus action can be dramatically enhanced because of rapid occurrence of clearance of HCV or HBV from blood;
2) it is anticipated that a binding constant of an oligopeptide added to HCV and interferon is not so high; also, even if a cell is infected via HCV E2 antigen to which C-terminal added and N-terminal added interferons are not bound, C-terminal added and N-terminal added interferons are present in a large amount in the surrounding of an infected cell; since C-terminal added and N-terminal added interferons have far higher affinity to an interferon receptor than to virus, enhancement of the local interferon action can be expected; and
3) by using C-terminal added and N-terminal added interferons bound to HCV or HBV, even if interferon disappears from blood after the administration, the interferon action easily sustains as long as virus bound to C-terminal added and N-terminal added interferons exist.

In addition, although it is considered that interferon with its terminal modified easily retains the original activity, there is a possibility that a new antigenicity becomes problematic. There is a possibility that the antigenicity is produced due to an amino acid sequence for a part for connecting interferon with an added oligopeptide. However, in the tertiary structure, it is deduced that addition of an oligopeptide to a terminal does not produce a great change in a tertiary structure. Therefore, since an added sequence has extremely high homologies with a human sequence, it is deduced that the antigenicity of a sequence itself is extremely low.

The fusion interferon of the present invention can be applied to any virus as long as a disease is a viral disease with which the interferon exhibits an anti-virus activity, in addition to human hepatitis C or B virus disease. Also, the fusion interferon can be applied to mammals such as ducks, chickens, horses, cows, sheep, dogs, cats and the like besides human.

The present invention will be described in further detail by means of examples, without intending to limit the present invention thereto.

### Example 1 Analysis of Homologous Region

### (1) Studies on Homologous Region of Human CD81, and Human and Bovine Lactoferrins

Based on nucleotide sequences of human CD81 (accession No. NM004356), bovine lactoferrin (accession No. M63502), and human lactoferrin (accession No. NM002343) genes, amino acid sequences were deduced from open reading frames contained therein. Each of the amino acid sequences is shown in SEQ ID NOs: 7 to 9.

The amino acid sequence homologies between human CD81 and bovine lactoferrin were obtained. As a result, the homologies were found between a region of 151st amino acid residue to 195th amino acid residue of CD81 (amino acid NOs: 151 to 195 of SEQ ID NO: 7) and a region of 439th amino acid residue to 481st amino acid residue of bovine lactoferrin (amino acid NOs: 439 to 481 of SEQ ID NO: 8). A region for which the homologies were found is shown in Figure 2. In Figure 2, Panel A of the sequence shows human-derived CD81 and Panel B shows bovine lactoferrin.

In addition, the homologies of amino acid sequences between bovine lactoferrin and human lactoferrin were studied. As a result, it was clarified that the sequence of 439th amino acid residue to 481st amino acid residue of bovine lactoferrin (amino acid NOs: 439 to 481 of SEQ ID NO: 8) corresponded to a sequence of 442nd amino acid residue to 484th amino acid residue of human lactoferrin (amino acid NOs: 442 to 484 of SEQ ID NO: 9), showing the high homologies between bovine-derived lactoferrin and human-derived lactoferrin. The analytical results on a homologous region are shown in Figure 3. In the sequence of Figure 3, an upper row shows bovine-derived lactoferrin, and a lower row shows human-derived lactoferrin. In the figure, a region for which the homologies with CD81 were exhibited is indicated.

A secondary structure of a partial amino acid sequence of each protein to which HCV is considered to bind was predicted. The results were shown in Figure 4. The homologous sequences between human CD81 and bovine lactoferrin are indicated in the figure. In the homologous sequences, the identical amino acids were indicated by bold characters, and the homologous amino acids were indicated by plain characters. In addition, in the prediction of the secondary structure, portions corresponding to amino acids constituting the same secondary structure between human CD81 and bovine lactoferrin were shaded with meshed patterns. In the case of human lactoferrin, the same portions as amino acids keeping a common secondary structure between a homologous sequence with human CD81, human CD81 and bovine lactoferrin in the amino acid sequence were shaded with meshed patterns.

As shown in Figure 4, it was found that the three sequences have the extremely similar structures except that in a homologous region, a structure of 450th amino acid residue to 453rd amino acid residue of human lactoferrin (amino acid NOs: 450 to 453 of SEQ ID NO: 9) takes Sheet structure, while the structures of those in human CD81 and bovine lactoferrin take Turn structure.

In addition, as shown in Figure 10, a sequence of human CD81 and sequences of bovine and human lactoferrins were compared. As a result, it was clarified that two cysteine residues existed in similar positions. The cysteine residues were positioned such that 33 amino acids were flanked by the cysteine residues in human CD81, and the cysteine residues were positioned such that 33 amino acids were flanked by the cysteine residues in bovine and human lactoferrins. This means that these regions have a common tertiary structure. It is considered that loop structure is kept by disulfide bond between the cysteine residues. Further, there was found the presence of basic amino acids consisting of arginine and lysine at a middle part of this loop structure and in the neighborhood of cysteine residue on C-terminal side. It is considered that the above-mentioned structural motif is important for binding of HCV. This shows that forming loop structure can increase the binding activity for virus by 1) forcibly generating disulfide bond in an oligopeptide, or 2) connecting N-terminal and C-terminal of an oligopeptide with a compound.

### (2) Studies on Homologous Region Between Human CD81 and LDL Receptor

Based on a nucleotide sequence of a human LDL receptor (accession No. NM000527) gene, an amino acid sequence was deduced from an open reading frame existing in the above-mentioned gene. An amino acid sequence excluding a signal sequence is shown in SEQ ID NO: 22.

The amino acid sequence homologies between human CD81 and human LDL receptor were obtained. As a result, the homologies were found between a region of 150th amino acid residue to 191st amino acid residue of human CD81 (amino acid NOs: 150 to 191 of SEQ ID NO: 7) and a region of 170th amino acid residue to 210th amino acid residue of a human LDL receptor (amino acid NOs: 170 to 210 of SEQ ID NO: 22). A region for which the homologies were found is shown in Figure 11. In the sequence of Figure 11, an upper row shows human-derived CD81, and a lower row shows a human LDL receptor. Portions shaded with meshed patterns in the amino acid sequence of CD81 are amino acid residues having the homologies with bovine and human lactoferrins, and portions shaded with meshed patterns of the amino acid sequence of a human LDL receptor are regions where the homologies were conserved.

In addition, the homologies between an amino acid sequence of human lactoferrin and that of a human LDL receptor were studied. As a result, the homologies were found between a region of 442nd amino acid residue to 484th amino acid residue of human lactoferrin (amino acid NOs: 442 to 484 of SEQ ID NO: 9) and a region of 196th residue to 239th residue of a human LDL receptor (amino acid NOs: 170 to 210 of SEQ ID NO: 22). A region for which the homologies were found is shown in Figure 12. As to the amino acid sequences shaded with meshed patterns, they are as defined in Figure 11.

The results of comparison of selected two kinds of human LDL receptor sequences and a homologous region between human CD81, and bovine and human lactoferrins are shown in Figure 10. As shown in Figure 10, it can be seen that cysteine residues in both sequences of two kinds of human LDL receptor sequences exist at similar positions with those of human CD81, and bovine and human lactoferrins. In addition, a region flanked by the cysteine residues was studied. As a result, it is shown that a region having the homologies with the human lactoferrin sequence is more consistent with a motif. However, since basic amino acids also exist in the neighborhood of cysteine residues on C-terminal side in the sequence selected by the homologies with human CD81, possibilities of the selected sequence to bind are suggested.

### Example 2 Studies on Binding Affinity to HCV E2 Region

An oligopeptide is synthesized in accordance with each amino acid sequence of homologous regions of human CD81, bovine lactoferrin and human lactoferrin. The resulting oligopeptide is coated on an ELISA plate in accordance with a conventional method. Next, a binding reaction is carried out by adding thereto HCV E2 antigen of Austral Biologicals, Inc. which can be purchased from Cosmo Bio.

As a control, an ELISA plate is coated with bovine lactoferrin protein of SERVA Electrophoresis GmbH. which can be purchased from Cosmo Bio, or human lactoferrin protein of Cappel Product.

After binding with the E2 protein and washing, an E2 antigen bound to an oligopeptide coated on a plate is detected by using a monoclonal antibody for the HCV E2 antigen of Austral Biologicals, Inc. which can be purchased from Cosmo Bio.

### Example 3 Selection of Amino Acid Sequence Binding to HCV

An oligopeptide consisting of about 20 amino acids is synthesized so as to overlap with the oligopeptide sequence of which binding activity to HCV E2 antigen in Example 2 is found. In accordance with Example 2, a region having the highest binding activity to the HCV E2 antigen may be selected, and an binding sequence may be further narrowed down. A binding experiment is carried out in the same manner, whereby a smallest oligonucleotide sequence which can bind to HCV E2 antigen can be identified.

### Example 4 Studies on Inhibition of Binding of CD81 to HCV

An oligopeptide capable of binding to HCV E2 antigen is reacted with the HCV E2 antigen to study whether or not binding of CD81 expressed in Jurkat cells purchased from DAINIPPON PHARMACEUTICAL CO., LTD. to the HCV E2 antigen is inhibited. By the study, whether or not the selected oligopeptide inhibits adhesion of the HCV E2 antigen to CD81-positive cells can be estimated. The inhibitory activity for adhesion of CD81 to the HCV E2 antigen was assayed in accordance with the literature. The concrete description will be given hereinbelow.

### (1) Confirmation of CD81 Expression Using FACScan

Jurkat cells (ATCC TIB-152) collected from a culturing T-75 flask (Coaster) in accordance with the conventional method were washed with centrifugation twice with 10 mL of Wash Buffer [phosphate-buffered saline (manufactured by Lifetech Oriental) containing 1% fetal bovine serum (manufactured by BIO WHITTAKER) and 0.1% sodium azide (manufactured by nakalai tesque); hereinafter simply referred to as WB].

Washed Jurkat cells were suspended in WB so as to have a concentration of 4 × 10⁶ cells/mL to give a Jurkat cell suspension. Twenty microliters of an anti-human CD81 antibody (BD PharMingen) was added to a 6 mm round-bottom tube (manufactured by Falcon). Further, 50 µL of the above-mentioned Jurkat cell suspension was added thereto, to give an anti-human CD81 antibody-Jurkat cell mixture.

In order to confirm the specificity of staining, 1 µg of a mouse IgG1 antibody (manufactured by BD PharMingen), the same isotype as the anti-human CD81 antibody, was added to a 6 mm round-bottom tube (manufactured by Falcon). Further, 50 µL of the above-mentioned Jurkat cell suspension was added thereto, to give a mouse IgG1 antibody-Jurkat cell mixture.

Each of the resulting anti-human CD81 antibody-Jurkat cell mixture and the mouse IgG1 antibody-Jurkat cell mixture was stirred, and each of these mixtures were then allowed to stand on ice for 30 minutes. Thereafter, both mixtures were washed with centrifugation twice with 2 mL of WB, and thereafter suspended in 0.1 mL of WB containing 1 µg of the FITC-labeled anti-mouse IgG1 antibody (manufactured by BD PharMingen). Each of the resulting suspensions was stirred, and thereafter allowed to stand on ice for 30 minutes. Subsequently, the resulting product was washed with centrifugation twice with 2 mL of WB, and thereafter suspended in 0.2 mL of a 10% neutral buffered formalin solution (manufactured by nakalai tesque). The resulting aliquots were analyzed by using FACScan (manufactured by Nihon Becton Dickinson). The above results are shown in Figure 13. In Panel (1) of Figure 13, when stained with the anti-human CD81 antibody, the FITC fluorescent intensity became stronger, as compared to that stained with the mouse IgG1. The results show that CD81 is expressed on the surface of the Jurkat cells. On the other hand, in Panel (2), the FITC fluorescent intensity was increased only when the Jurkat cells which had been reacted with the recombinant E2 protein, were stained with the anti-E2 antibody. Therefore, it is shown that the recombinant E2 protein is bound to the Jurkat cells.

As a result, as shown in Figure 13, it can be seen that CD81, which is a receptor for HCV E2 protein, is expressed on almost all the Jurkat cells.

### (2) Analysis on Binding of Recombinant E2 Protein to Jurkat Cells

The Jurkat cells were prepared in the same manner as in item (1), and suspended in WB so as to have a concentration of 4 × 10⁶ cells/mL, to give a Jurkat cell suspension. One microgram of the recombinant E2 protein (Austral Biologicals) was added to a 6 mm rounded-bottom tube, and 50 µL of the Jurkat cell suspension was added thereto, to give a recombinant E2 protein-Jurkat cell mixture.

The above-mentioned recombinant E2 protein-Jurkat cell mixture was stirred and thereafter allowed to stand on ice for 30 minutes. The above-mentioned mixture was washed with centrifugation twice with 2 mL of WB, and thereafter suspended in 0.1 mL of WB containing 1 µg of an anti-E2 antibody (Austral Biologicals). After stirring, the mixture was allowed to stand on ice for 30 minutes.

Hereinafter, in the same manner as in the item (1) above, the resulting product was reacted with an FITC-labeled anti-mouse IgG1 antibody, and thereafter the reaction mixture was suspended in 0.2 mL of a 10% neutral buffered formalin solution. As to the resulting product, E2 protein bound to Jurkat cells was analyzed by using FACScan. The results are shown in Figure 13.

The FITC fluorescent intensity which had been increased by the addition of the untreated recombinant E2 protein to the Jurkat cells was weakened by the reaction of a peptide or protein with the recombinant E2 protein. This shows that the peptide or the protein inhibited the binding activity of the recombinant E2 protein to the Jurkat cells.

Accordingly, as shown in Figure 13, it can be seen that the E2 protein is bound to the Jurkat cells.

### (3) Peptide Synthesis

Peptides having amino acid sequences of SEQ ID NOs: 23 to 28 were synthesized on the solid phase by Fmoc method, and purified by reverse phase HPLC. Each of the amino acid sequences shows the following peptides:
SEQ ID NO: 23 Human CD81-derived peptide: TAPA148,
SEQ ID NO: 24 Bovine lactoferrin-derived peptide: bLF443,
SEQ ID NO: 25 Human lactoferrin-derived peptide: hLF445,
SEQ ID NO: 26 Human LDL receptor-derived peptide: hLDLR194,
SEQ ID NO: 27 Human LDL receptor-derived peptide: hLDLR220, and
SEQ ID NO: 28 Bovine lactoferrin-derived cyclic peptide: cbLF445.

Cyclization of the above-mentioned bovine lactoferrin-derived cyclic peptide of SEQ ID NO: 28 was carried out by forming a disulfide bond with cysteine residues positioned at N-terminal and C-terminal. Further, the cyclization was confirmed by mass spectroscopy of the purified peptide.

### (4) Preparation of Peptide Solution

The peptide obtained in the item (3) above was dissolved in a phosphate buffered saline (hereinafter, referred to as PBS; manufactured by Nissuiseiyaku) so as to have a concentration of 1 mg/mL. In addition, bovine lactoferrin (SERVA Electrophoresis GmbH) was dissolved in PBS so as to have a concentration of 1 mg/mL. Thereafter, each of the resulting solutions was sterilized by filtration using a 0.2 µm cartridge filter (Millipore Corporation). Each of the resulting filtrates was dispensed in an amount of 0.2 mL of and frozen-stored at 80°C. The resulting solutions are referred to as a peptide solution and a purified bovine lactoferrin solution.

### (5) Studies on Inhibitory Action of Binding of E2 by Peptide

One microgram of the recombinant E2 protein, and 10 µL of the TAPA148, bLF or bLF443 solution obtained in item (4) above were added to a 6 mm rounded-bottom tube and, and WB was then added thereto, to give 50 µL of a mixture. The resulting mixture was stirred and allowed to stand at room temperature for 1 hour. Fifty microliters of the Jurkat cell suspension (4 × 10⁶ cells/mL) described in item (1) above was added to the resulting solution, and the mixture was stirred and then allowed to stand on ice for 30 minutes. Hereinafter, in the same manner as in item (2) above, the resulting solution was reacted successively with an anti-E2 antibody and an FITC-labeled anti-mouse IgG1 antibody, and the reaction mixture was suspended in 0.2 mL of a 10% neutral buffered formalin solution. As to the resulting suspension, E2 protein bound to Jurkat cells was analyzed by using FACScan.

As shown in Figure 14, there is found the inhibitory action of binding of E2 protein by the human CD81-derived peptide (TAPA148). In addition, there is likewise found the inhibitory action of binding of the E2 protein by the bovine lactoferrin (bLF). Further, there is likewise found the inhibitory action of binding of the E2 protein when the bovine lactoferrin-derived peptide (bLF443) is used.

### Example 5 Preparation of Interferon to Which HCV-Bound Oligopeptide Is Added

A complementary nucleotide sequence having about 20 bases, immediately before a termination codon of interferon-α2b, and a DNA encoding the selected oligopeptide are fused, and a termination codon sequence was further connected to 3'-terminal, and there are further synthesized 3'-side PCR primers having a restriction enzyme digesting sequence so that the sequence can be cloned into an appropriate restriction enzyme site of pBK-CMV, which can be purchased from Stratagene. In addition, there is synthesized a DNA having a restriction enzyme digesting sequence at a 5'-terminal as a 5'-side primer, and containing a 5'-upstream region of interferon-α2b and a homologous nucleotide sequence having about 20 bases. Thereafter, reaction of PCR is carried out by using a cDNA clone of interferon-α2b obtained with Namalwa cells which can be purchased from DAINIPPON PHARMACEUTICAL CO., LTD. as a template. The resulting PCR amplified fragment is digested with a restriction enzyme, and then inserted into pBK-CMV, to give an *Escherichia coli* transformant in accordance with a conventional method. A recombinant plasmid harboring the above-mentioned PCR-amplified fragment is isolated from the resulting transformant. Next, appropriate cells (for example, COS-1 cells, CHO cells etc.) are transfected with the resulting recombinant plasmid by calcium phosphate method, to give C-terminal added interferon-α2b. The resulting culture supernatant is added to various human-derived cell strains (HeLa cells, HepG2 cells and the like), whereby confirming that 2-5A synthase is induced by C-terminal added interferon-α2b. In addition, the resulting C-terminal added interferon-α2b and HCV E2 antigen are incubated, and it is confirmed that adhesion of CD81 expressed in the Jurkat cells to HCV E2 antigen is inhibited by C-terminal added interferon-α2b in accordance with the method described in Example 4. The concrete description will be given hereinbelow.

It is noted that, in the present experiment, the C-terminal added interferon-α2b was produced using COS-1 cells (purchased from The Institute of Physical and Chemical Research).

### (1) Cloning of Human Interferon-α2b Gene by PCR and Preparation of Interferon-α2b Expression Vector

PCR was carried out by using KOD Plus DNA polymerase (TOYOBO CO., LTD.) and interferon-α2b-specific primers U-2 (SEQ ID NO: 29) and L-2 (SEQ ID NO: 30), with human genome DNA (manufactured by Promega; cat#: G304A, lot#: 9273102L) as a template. In PCR, there was used the following reaction solution [a solution prepared by mixing 988 ng of human genome DNA, 10 µl of KOD Plus DNA polymerase 10 × reaction buffer, 10 µl of dNTPs (2 mM each), 4.8 µl of MgSO₄ (25 mM), 5 µl of primer U-2 (0.1 µg/µl), 5 µl of primer L-2 (0.1 µg/µl) and 2 µl of KOD Plus DNA polymerase (1 U/µl), and adding distilled water thereto to make up a volume of 100 µl].

As a thermal cycler, GeneAmp 9700 (manufactured by Perkin-Elmer) was used. The thermal profile comprises 1 cycle of 95°C-3 minutes, 30 cycles of (95°C-20 seconds, 60°C-30 seconds, and 72°C-3 minutes), and 1 cycle of 72°C-7 minutes, and thereafter, the reaction mixture was allowed to stand at 4°C overnight.

The resulting PCR product was purified using QIAGEN PCR Purification Kit in accordance with the attached instruction. The purified DNA fragment was digested with BamHI and HindIII, and the resulting product was purified. In addition, pcDNA3.1(-) (manufactured by Invitrogen) was digested with BamHI and HindIII, and the resulting product was purified. A DNA fragment was inserted into BamHI and HindIII site of the resulting pcDNA3.1(-) digest. By the above procedures, the IFN-α2b expression vector (plasmid was named pCIA) was prepared. In addition, the nucleotide sequence was read and compared with GenBank accession #J00207, V00544, and confirmed to be correct.

### (2) Preparation of HCV-Binding type IFNs Expression Vectors

An expression vector for a protein, in which human CD81, bovine lactoferrin or a deduced HCV E2 binding region is fused to C-terminal of human IFN-α2b (hereinafter, referred to as "HCV-binding type IFNs") was prepared by the following procedures 1), 2) and 3) (Figure 15).

### 1) Introduction of HindIII Site as Connecting Site into C-Terminal of IFN-α2b by PCR

PCR was carried out by using T7 promoter primer and H3TER primer (SEQ ID NO: 31) with the IFN-α2b expression vector pCIA as a template, to give a DNA fragment in which HindIII site is introduced into a nucleotide sequence corresponding to C-terminal of IFN-α2b, and thereafter the DNA fragment was inserted into pcDNA3.1(-)(plasmid was named pCIA-H). The HindIII site was made so that an amino acid sequence of IFN-α2b was not changed.

### 2) Preparation of Deduced HCV E2 Binding Regions of Each of CD81, Bovine Lactoferrin and Human Lactoferrin by Synthetic DNA

A synthetic DNA of a deduced HCV E2 binding region of each of CD81, bovine lactoferrin and human lactoferrin was prepared, and the DNA was cloned into pUC18. The deduced HCV E2 binding region of CD81 is shown in SEQ ID NO: 32; the deduced HCV E2 binding region of bovine lactoferrin is shown in SEQ ID NO: 33; and the deduced HCV E2 binding region of human lactoferrin is shown in SEQ ID NO: 34. A nucleotide sequence of each of the resulting clones was confirmed by sequencing.

### 3) Ligation of IFN-α2b Gene with Each of Deduced HCV E2 Binding Regions of CD81, Bovine Lactoferrin and Human Lactoferrin

There was prepared a vector in which a HindIII fragment having 564 bp of λ phage as a stuffer sequence is once inserted into HindIII site of pCIA-H. The resulting vector was digested with AfIII and then digested with HindIII. Into the resulting product was inserted each of the DNA fragments encoding deduced HCV E2 binding regions of CD81, bovine lactoferrin and human lactoferrin, the DNA fragments resulting from digestion from the plasmid prepared in item 2) above with HindIII and AfIII. A nucleotide sequence of each of the resulting clones was confirmed by sequencing.

By the above procedures, there were prepared expression vectors (HCV-binding type IFN expression vectors) pCIA-CD81, pCIA-BLFN and pCIA-HLFN, resulting from fusion of each of deduced HCV E2 binding regions of human CD81, bovine lactoferrin and human lactoferrin to C-terminal of human IFN-α2b.

### (3) Protein Synthesis by in vitro Transcription/Translation and Western Blot Analysis of IFN-α

Without using a radioactive isotope, protein synthesis was carried out by *in vitro* transcription/translation using TNT T7 coupled wheat germ extract system (manufactured by Promega), with pCIA, pCIA-CD81, pCIA-BLFN and pCIA-HLFN as a template. The resulting products were subjected to 15% SDS-PAGE. Thereafter, the proteins in the gel after electrophoresis were transferred to a membrane (Immobillon P, manufactured by Millipore Corporation). Western blot analysis was carried out by using the resulting membrane and an anti-IFN-α monoclonal antibody (clone MMHA-2, manufactured by PBL Biomedical). Here, the blocking in Western blot was carried out by using a blocking buffer [TBS-T (composition: 50 mM Tris-HCl (pH 7.5), 15 mM NaCl, 0.05% Tween 20) containing 3% normal goat serum (Vector)]. In addition, a dilution prepared by 1000-fold-dilution of the anti-IFN-α monoclonal antibody with a blocking buffer was used as a primary antibody, and a dilution obtained by 5000-fold-dilution of a peroxidase-labeled anti-mouse IgG goat antibody (manufactured by BIOSOURCE INTERNATIONAL) with a blocking buffer was used as a secondary antibody. Detection was made by using ECL Plus (manufactured by Amersham Pharmacia).

As shown in Figure 16, it can be seen that IFN-α2b and HCV-binding type IFNs are synthesized with each plasmid as a template.

### (4) Transient Expression in COS-1 Cells

COS-1 cells were transfected with each of pCIA, pCIA-CD81, pCIA-BLFN and pCIA-HLFN by using FuGENE6 (Roche Diagnostics GmbH). The culture after the transfection was carried out on DMEM medium without addition of an antibiotic and without addition of serum. Three days after the transfection, the culture supernatants were collected. To each of the culture supernatants, fresh medium was added, and the culture was continued. Six days after the transfection, the culture supernatants were collected.

The amount of the recombinant proteins produced in the culture supernatants were evaluated by using IFN-αELISA kit (manufactured by BIOSOURCE INTERNATIONAL). The results are shown in Table 1.

In addition, the recombinant proteins in the culture supernatants was subjected to Western blot analysis in the manner described above by using an anti-IFN-α monoclonal antibody (clone MMHA-2, PBL Biomedical). As shown in Figure 17, it can be seen that each of IFN-α2b, IFN-α-CD81 fusion protein, IFN-α-bovine lactoferrin fusion protein and IFN-α-human lactoferrin fusion protein was detected in the culture supernatant of COS-1 cells transfected with each of pCIA, pCIA-CD81, pCIA-BLFN and pCIA-HLFN. By the above procedures, it was confirmed that IFN-α2b and HCV-binding type IFNs were transiently expressed in COS-1 cells. The interferon concentration in the resulting culture supernatants is shown in Table 1. When COS-1 cells were transfected with an empty vector pcDNA 3.1 to assay an anti-virus activity (described below), no anti-virus activity was found in the culture supernatant, so that it was confirmed that endogenous interferon is not induced.

**Table 1**

| Interferon Concentration in Culture Supernatant of Transfected COS-1 Cells | | |
|---|---|---|
| Vector | Day 3 | Day 6 |
| pCIA | 936 ng/ml | 173 ng/ml |
| pCIA-BLFN | 654 ng/ml | 218 ng/ml |
| pCIA-CD81 | 976 ng/ml | 299 ng/ml |
| pCIA-HLFN | 258 ng/ml | 110 ng/ml |

### (5) Anti-Virus Activity of Recombinant Protein

The anti-virus activities of IFN-α2b and HCV-binding type IFNs which had been prepared from transiently expressed in COS-1 cells were assayed by an assay system using the system for evaluating Sindbis virus-FL cells. Each of 200 IU/mL human interferon α2b (INTRON A, manufactured by Sherring-Plough/YAMANOUCHI PHARMACEUTICAL CO., LTD.) or the culture supernatants of COS-1 cells resulting from transient expression of IFN-α2b and HCV-binding type IFNs was two-fold serial dilution with a medium containing fetal bovine serum where 1000-fold dilution was regarded as a dilution fold 1. The resulting dilution was cultured together with the FL cells.

On the next day, Sindbis virus was added to the resulting culture, and the mixture was cultured for 2 days. Thereafter, the absorbance of the resulting culture at 595 nm was determined by Crystal Violet method. The determination was taken at n = 2. In addition, at the same time, the FL cells alone were cultured as cell control, and the untreated FL cells added with Sindbis virus were cultured as virus control. The results are shown in Figure 18 and Figure 19.

It was confirmed from these results that IFN-α2b and HCV-binding type IFNs which had been transiently expressed in COS-1 cells have the anti-virus activity.

### (6) Binding of HCV-Binding type IFNs to HCV E2 Protein

There was evaluated the binding of IFN-α2b or HCV-binding type IFNs which had been transiently expressed in COS-1 cells to the baculovirus-derived recombinant GST-fusion HCV E2 protein (hereinafter referred to as GST-E2; manufactured by Immuno Diagnostics) by coprecipitation using Glutathione beads. Five microliters of GST-E2 (1 µg/µl) was mixed with 400 µl of the culture supernatant of COS-1 cells containing transiently expressed IFN-α2b or HCV-binding type IFNs, and were incubated at 4°C for 1 hour. Glutathione Sepharose 4B (Amersham Pharmacia) which had been incubated in buffer TBS-X [composition: 50 mM Tris-HCl (pH 7.5), 150 mM NaCl, 0.1% Triton X-100] containing 3% BSA for 30 minutes was added to a mixture of the culture supernatant of COS-1 cells and GST-E2, and the aliquots were incubated overnight at 4°C. After centrifugation, the supernatants (non-binding fraction) were collected, and the precipitated Glutathione beads were washed five times with the above-mentioned TBS-X. A 2 x sample buffer was added to a bound fraction Glutathione beads, and the aliquots were treated at 100°C for 5 minutes. Thereafter, the supernatants obtained by centrifugation were subjected to Western blot analysis using an anti-IFN-α antibody. Similarly, the supernatants of the non-binding fraction were subjected to Western blot analysis. The Western blot analysis was carried out in the same manner as in item (3) above. As shown in Figure 20, HCV-binding type IFNs were coprecipitated with GST-E2, but IFN-α2b was not. It was suggested from these results that HCV-binding type IFNs bind to GST-E2 although IFN-α2b itself does not bind to GST-E2.

### Example 6 Identification of Binding Sequence of HBV to pre-S

A binding region was analyzed, on the bases of an amino acid sequence of duck-derived carboxypeptidase D of SEQ ID NO: 14, and an amino acid sequence of duck-derived glycine decarboxylase complex P protein of SEQ ID NO: 15, which had been reportedly bound to duck HBV pre-S antigen. In the case of duck-derived carboxypeptidase D, 25 amino acids including N-terminal signal sequence and a region of 1307th amino acid residue to 1333rd amino acid residue (amino acid NOs: 1307 to 1333 in the amino acid sequence disclosed in accession No. AF039749) have been considered to be important for binding to pre-S. In addition, in the duck-derived glycine decarboxylase complex P protein, a sequence consisting of 52nd amino acid residue to 94th amino acid residue (amino acid NOs: 52 to 94 in the amino acid sequence disclosed in accession No. AF137264), and a sequence consisting of 923rd amino acid residue to 1024th amino acid residue, the C-terminal (amino acid NOs: 923 to 1024 disclosed in the above-mentioned accession No. AF137264) are considered to be necessary for binding. Therefore, the homologies between the amino acid sequences of respective regions were studied. The results are shown in Figure 5. In the study of the homologies of N-terminal side sequence, a homologous region 1, which is thought to include a signal peptide of carboxypeptidase D was selected. However, since a signal sequence usually consists of 11 to 13 amino acids, the homologous region 1 is hardly likely to be a binding region for HBV receptor. Probably, the LLP sequence of a homologous region 2 is thought to be important. In addition, in the analysis on C-terminal side, the homologies were found in a region consisting of 1313th amino acid residue to 1333rd amino acid residue of carboxypeptidase D (amino acid NOs: 1313 to 1333 in the amino acid sequence disclosed in accession No. AF039749) and a region consisting of 943rd amino acid residue to 963rd amino acid residue of glycine decarboxylase complex P protein (amino acid NOs: 943 to 963 in the amino acid sequence disclosed in accession No. AF137264). Next, when a secondary structure of each homologous region was predicted, especially in the analysis of a similar region on C-terminal side, it was clarified that only in the region where identical amino acids are present in a large number takes the same tertiary structure. The results of prediction of the secondary structure are shown in Figure 6.

A binding region to duck HBV obtained in each duck-derived gene was compared with an amino acid sequence of human-derived carboxypeptidase D of SEQ ID NO: 16 and an amino acid sequence of human-derived glycine decarboxylase complex P protein of SEQ ID NO: 17. The evaluation results for homologies of each of the genes of duck and human are shown in Figure 7, Figure 8 and Figure 9. Although the high homologies were not found in a region which is thought to be an HBV-binding region of each carboxypeptidase D, other regions were well conserved in duck and human genes. Binding deduced regions for HBV are shown in Figure 7, Figure 8 and Figure 9. It is deduced from the results that a region acquiring the affinity for HBV is C-terminal side sequence.

### SEQUENCE LISTING FREE TEXT

SEQ ID NO: 23 shows a sequence of a synthesized peptide of human CD81-derived peptide TAPA148.

SEQ ID NO: 24 shows a sequence of a synthesized peptide of bovine lactoferrin-derived peptide bLF443.

SEQ ID NO: 25 shows a sequence of a synthesized peptide of human lactoferrin-derived peptide hLF445.

SEQ ID NO: 26 shows a sequence of a synthesized peptide of human LDL receptor-derived peptide hLDLR194.

SEQ ID NO: 27 shows a sequence of a synthesized peptide of human LDL receptor-derived peptide hLDLR220.

SEQ ID NO: 28 shows a sequence of a synthesized peptide of bovine lactoferrin-derived cyclic peptide cbLF445.

SEQ ID NO: 29 shows a sequence of a synthesized oligonucleotide for interferon-α2b-specific primer U-2.

SEQ ID NO: 30 shows a sequence of a synthesized oligonucleotide for interferon-α2b-specific primer L2.

SEQ ID NO: 31 shows a sequence of a synthesized oligonucleotide for H3TER primer.

SEQ ID NO: 32 shows a sequence of a synthesized nucleotide having a sequence corresponding to the deduced HCV E2 binding region in CD81.

SEQ ID NO: 33 shows a sequence of a synthesized nucleotide having a sequence corresponding to the deduced HCV E2 binding region in bovine lactoferrin.

SEQ ID NO: 34 shows a sequence of a synthesized nucleotide having a sequence corresponding to the deduced HCV E2 binding region in human lactoferrin.

### INDUSTRIAL APPLICABILITY

According to the prophylactic or therapeutic agent for chronic hepatitis of the present invention, it is considered that the binding of hepatitis virus, especially hepatitis C virus or hepatitis B virus, to a target cell is inhibited. Therefore, there are exhibited excellent effects that chronic hepatitis can be prevented or treated. Therefore, in the interferon treatment method for chronic type-C and type-C hepatitis, there are even more expected that the viral clearance ratio at the termination of the interferon administration can be improved by the suppression of the infection on a target cell for hepatitis virus. The improvement in the clearance ratio of the hepatitis virus at the termination of the interferon according to the present invention is clinically very effective, from the viewpoints such that the improvement not only leads to an increase in complete responders but also that the development of hepatocellular carcinoma can be suppressed.

SEQUENCE LISTING

<110> SUMITOMO PHARMACEUTICALS COMPANY, LIMITED

<120> Prophylactic agents and therapeutic agents for chronic hepatitis.

<130> 00-065-PCT

<150> JP 11-374087

<151> 1999-12-28

<160> 34

<210> 1

<211> 45

<212> PRT

<213> Homo sapiens

<400> 1

His Glu Thr Leu Asp Cys Cys Gly Ser Ser Thr Leu Thr Ala Leu Thr

1               5                   10                  15

Thr Ser Val Leu Lys Asn Asn Leu Cys Pro Ser Gly Ser Asn Ile Ile

            20                  25                  30

Ser Asn Leu Phe Lys Glu Asp Cys His Gln Lys Ile Asp

        35                  40                  45

<210> 2

<211> 43

<212> PRT

<213> Bovine

<400> 2

His Ser Ser Leu Asp Cys Val Leu Arg Pro Thr Glu Gly Tyr Leu Ala

1               5                   10                  15

Val Ala Val Val Lys Lys Ala Asn Glu Gly Leu Thr Trp Asn Ser Leu

            20                  25                  30

Lys Asp Lys Lys Ser Cys His Thr Ala Val Asp

        35                  40

<210> 3

<211> 43

<212> PRT

<213> Homo sapiens

<400> 3

Asp Pro Asp Pro Asn Cys Val Asp Arg Pro Val Glu Gly Tyr Leu Ala

1               5                   10                  15

Val Ala Val Val Arg Arg Ser Asp Thr Ser Leu Thr Trp Asn Ser Val

            20                  25                  30

Lys Gly Lys Lys Ser Cys His Thr Ala Val Asp

        35                  40

<210> 4

<211> 135

<212> DNA

<213> Homo sapiens

<400> 4

cacgagacgc ttgactgctg tggctccagc acactgactg ctttgaccac ctcagtgctc 60

aagaacaatt tgtgtccctc gggcagcaac atcatcagca acctcttcaa ggaggactgc 120

caccagaaga tcgat 135

<210> 5

<211> 129

<212> DNA

<213> Bovine

<400> 5

cacagtagcc tagattgtgt gctgagacca acggaagggt accttgccgt ggcagttgtc 60

aagaaagcaa atgaggggct cacatggaat tctctgaaag acaagaagtc gtgccacacc 120

gccgtggac 129

<210> 6

<211> 129

<212> DNA

<213> Homo sapiens

<400> 6

gaccctgatc ctaactgtgt ggatagacct gtggaaggat atcttgctgt ggcggtggtt 60

aggagatcag acactagcct tacctggaac tctgtgaaag gcaagaagtc ctgccacacc 120

gccgtggac 129

<210> 7

<211> 236

<212> PRT

<213> Homo sapiens

<400> 7

Met Gly Val Glu Gly Cys Thr Lys Cys Ile Lys Tyr Leu Leu Phe Val

1               5                   10                  15

Phe Asn Phe Val Phe Trp Leu Ala Gly Gly Val Ile Leu Gly Val Ala

            20                  25                  30

Leu Trp Leu Arg His Asp Pro Gln Thr Thr Asn Leu Leu Tyr Leu Glu

        35                  40                  45

Leu Gly Asp Lys Pro Ala Pro Asn Thr Phe Tyr Val Gly Ile Tyr Ile

    50                  55                  60

Leu Ile Ala Val Gly Ala Val Met Met Phe Val Gly Phe Leu Gly Cys

65                  70                  75                  80

Tyr Gly Ala Ile Gln Glu Ser Gln Cys Leu Leu Gly Thr Phe Phe Thr

                85                  90                  95

Cys Leu Val Ile Leu Phe Ala Cys Glu Val Ala Ala Gly Ile Trp Gly

            100                 105                 110

Phe Val Asn Lys Asp Gln Ile Ala Lys Asp Val Lys Gln Phe Tyr Asp

        115                 120                 125

Gln Ala Leu Gln Gln Ala Val Val Asp Asp Asp Ala Asn Asn Ala Lys

    130                 135                 140

Ala Val Val Lys Thr Phe His Glu Thr Leu Asp Cys Cys Gly Ser Ser

145                 150                 155                 160

Thr Leu Thr Ala Leu Thr Thr Ser Val Leu Lys Asn Asn Leu Cys Pro

                165                 170                 175

Ser Gly Ser Asn Ile Ile Ser Asn Leu Phe Lys Glu Asp Cys His Gln

            180                 185                 190

Lys Ile Asp Asp Leu Phe Ser Gly Lys Leu Tyr Leu Ile Gly Ile Ala

        195                 200                 205

Ala Ile Val Val Ala Val Ile Met Ile Phe Glu Met Ile Leu Ser Met

    210                 215                 220

Val Leu Cys Cys Gly Ile Arg Asn Ser Ser Val Tyr

225                 230                 235

<210> 8

<211> 708

<212> PRT

<213> Bovine

<400> 8

Met Lys Leu Phe Val Pro Ala Leu Leu Ser Leu Gly Ala Leu Gly Leu

1               5                   10                  15

Cys Leu Ala Ala Pro Arg Lys Asn Val Arg Trp Cys Thr Ile Ser Gln

            20                  25                  30

Pro Glu Trp Phe Lys Cys Arg Arg Trp Gln Trp Arg Met Lys Lys Leu
        35                  40                  45
Gly Ala Pro Ser Ile Thr Cys Val Arg Arg Ala Phe Ala Leu Glu Cys
    50                  55                  60
Ile Pro Gly Ile Ala Glu Lys Lys Ala Asp Ala Val Thr Leu Asp Gly
65                  70                  75                  80
Gly Met Val Phe Glu Ala Gly Arg Asp Pro Tyr Lys Leu Arg Pro Val
                85                  90                  95
Ala Ala Glu Ile Tyr Gly Thr Lys Glu Ser Pro Gln Thr His Tyr Tyr
            100                 105                 110
Ala Val Ala Val Val Lys Lys Gly Ser Asn Phe Gln Leu Asp Gln Leu
        115                 120                 125
Gln Gly Arg Lys Ser Cys His Thr Gly Leu Gly Arg Ser Ala Gly Trp
    130                 135                 140
Ile Ile Pro Met Gly Ile Leu Arg Pro Tyr Leu Ser Trp Thr Glu Ser
145                 150                 155                 160
Leu Glu Pro Leu Gln Gly Ala Val Ala Lys Phe Phe Ser Ala Ser Cys
                165                 170                 175
Val Pro Cys Ile Asp Arg Gln Ala Tyr Pro Asn Leu Cys Gln Leu Cys
            180                 185                 190
Lys Gly Glu Gly Glu Asn Gln Cys Ala Cys Ser Ser Arg Glu Pro Tyr
        195                 200                 205
Phe Gly Tyr Ser Gly Ala Phe Lys Cys Leu Gln Asp Gly Ala Gly Asp
    210                 215                 220
Val Ala Phe Val Lys Glu Thr Thr Val Phe Glu Asn Leu Pro Glu Lys
225                 230                 235                 240

Ala Asp Arg Asp Gln Tyr Glu Leu Leu Cys Leu Asn Asn Ser Arg Ala
                245                 250                 255
Pro Val Asp Ala Phe Lys Glu Cys His Leu Ala Gln Val Pro Ser His
            260                 265                 270
Ala Val Val Ala Arg Ser Val Asp Gly Lys Glu Asp Leu Ile Trp Lys
        275                 280                 285
Leu Leu Ser Lys Ala Gln Glu Lys Ser Gly Lys Asn Lys Ser Arg Ser
    290                 295                 300
Phe Gln Leu Phe Gly Ser Pro Pro Gly Gln Arg Asp Leu Leu Phe Lys
305                 310                 315                 320
Asp Ser Ala Leu Gly Phe Leu Arg Ile Pro Ser Lys Val Asp Ser Ala
                325                 330                 335
Leu Tyr Leu Gly Ser Arg Tyr Leu Thr Thr Leu Lys Asn Leu Arg Glu
            340                 345                 350
Thr Ala Glu Glu Val Lys Ala Arg Tyr Thr Arg Val Val Trp Cys Ala
        355                 360                 365
Val Gly Pro Glu Glu Gln Lys Lys Cys Gln Gln Trp Ser Gln Gln Ser
    370                 375                 380
Gly Gln Asn Val Thr Cys Ala Thr Ala Ser Thr Thr Asp Asp Cys Ile
385                 390                 395                 400
Val Leu Val Leu Lys Gly Glu Ala Asp Ala Leu Asn Leu Asp Gly Gly
                405                 410                 415
Tyr Ile Tyr Thr Ala Gly Lys Cys Gly Leu Val Pro Val Leu Ala Glu
            420                 425                 430
Asn Arg Lys Ser Ser Lys His Ser Ser Leu Asp Cys Val Leu Arg Pro

        435                 440                 445
Thr Glu Gly Tyr Leu Ala Val Ala Val Val Lys Lys Ala Asn Glu Gly
    450                 455                 460
Leu Thr Trp Asn Ser Leu Lys Asp Lys Lys Ser Cys His Thr Ala Val
465                 470                 475                 480
Asp Arg Thr Ala Gly Trp Asn Ile Pro Met Gly Leu Ile Val Asn Gln
                485                 490                 495
Thr Gly Ser Cys Ala Phe Asp Glu Phe Phe Ser Gln Ser Cys Ala Pro
            500                 505                 510
Gly Ala Asp Pro Lys Ser Arg Leu Cys Ala Leu Cys Ala Gly Asp Asp
        515                 520                 525
Gln Gly Leu Asp Lys Cys Val Pro Asn Ser Lys Glu Lys Tyr Tyr Gly
    530                 535                 540
Tyr Thr Gly Ala Phe Arg Cys Leu Ala Glu Asp Val Gly Asp Val Ala
545                 550                 555                 560
Phe Val Lys Asn Asp Thr Val Trp Glu Asn Thr Asn Gly Glu Ser Thr
                565                 570                 575
Ala Asp Trp Ala Lys Asn Leu Asn Arg Glu Asp Phe Arg Leu Leu Cys
            580                 585                 590
Leu Asp Gly Thr Arg Lys Pro Val Thr Glu Ala Gln Ser Cys His Leu
        595                 600                 605
Ala Val Ala Pro Asn His Ala Val Val Ser Arg Ser Asp Arg Ala Ala
    610                 615                 620
His Val Lys Gln Val Leu Leu His Gln Gln Ala Leu Phe Gly Lys Asn
625                 630                 635                 640
Gly Lys Asn Cys Pro Asp Lys Phe Cys Leu Phe Lys Ser Glu Thr Lys

                645                 650                 655

Asn Leu Leu Phe Asn Asp Asn Thr Glu Cys Leu Ala Lys Leu Gly Gly

            660                 665                 670

Arg Pro Thr Tyr Glu Glu Tyr Leu Gly Thr Glu Tyr Val Thr Ala Ile

        675                 680                 685

Ala Asn Leu Lys Lys Cys Ser Thr Ser Pro Leu Leu Glu Ala Cys Ala

    690                 695                 700

Phe Leu Thr Arg

705

<210> 9

<211> 711

<212> PRT

<213> Homo sapiens

<400> 9

Met Lys Leu Val Phe Leu Val Leu Leu Phe Leu Gly Ala Leu Gly Leu

1               5                   10                  15

Cys Leu Ala Gly Arg Arg Arg Arg Ser Val Gln Trp Cys Ala Val Ser

            20                  25                  30

Gln Pro Glu Ala Thr Lys Cys Phe Gln Trp Gln Arg Asn Met Arg Lys

        35                  40                  45

Val Arg Gly Pro Pro Val Ser Cys Ile Lys Arg Asp Ser Pro Ile Gln

    50                  55                  60

Cys Ile Gln Ala Ile Ala Glu Asn Arg Ala Asp Ala Val Thr Leu Asp

65                  70                  75                  80

Gly Gly Phe Ile Tyr Glu Ala Gly Leu Ala Pro Tyr Lys Leu Arg Pro
                85                  90                  95
Val Ala Ala Glu Val Tyr Gly Thr Glu Arg Gln Pro Arg Thr His Tyr
            100                 105                 110
Tyr Ala Val Ala Val Val Lys Lys Gly Gly Ser Phe Gln Leu Asn Glu
        115                 120                 125
Leu Gln Gly Leu Lys Ser Cys His Thr Gly Leu Arg Arg Thr Ala Gly
    130                 135                 140
Trp Asn Val Pro Thr Gly Thr Leu Arg Pro Phe Leu Asn Trp Thr Gly
145                 150                 155                 160
Pro Pro Glu Pro Ile Glu Ala Ala Val Ala Arg Phe Phe Ser Ala Ser
                165                 170                 175
Cys Val Pro Gly Ala Asp Lys Gly Gln Phe Pro Asn Leu Cys Arg Leu
            180                 185                 190
Cys Ala Gly Thr Gly Glu Asn Lys Cys Ala Phe Ser Ser Gln Glu Pro
        195                 200                 205
Tyr Phe Ser Tyr Ser Gly Ala Phe Lys Cys Leu Arg Asp Gly Ala Gly
    210                 215                 220
Asp Val Ala Phe Ile Arg Glu Ser Thr Val Phe Glu Asp Leu Ser Asp
225                 230                 235                 240
Glu Ala Glu Arg Asp Glu Tyr Glu Leu Leu Cys Pro Asp Asn Thr Arg
                245                 250                 255
Lys Pro Val Asp Lys Phe Lys Asp Cys His Leu Ala Arg Val Pro Ser
            260                 265                 270
His Ala Val Val Ala Arg Ser Val Asn Gly Lys Glu Asp Ala Ile Trp
        275                 280                 285

Asn Leu Leu Arg Gln Ala Gln Glu Lys Phe Gly Lys Asp Lys Ser Pro
    290                 295                 300
Lys Phe Gln Leu Phe Gly Ser Pro Ser Gly Gln Lys Asp Leu Leu Phe
305                 310                 315                 320
Lys Asp Ser Ala Ile Gly Phe Ser Arg Val Pro Pro Arg Ile Asp Ser
                325                 330                 335
Gly Leu Tyr Leu Gly Ser Gly Tyr Phe Thr Ala Ile Gln Asn Leu Arg
            340                 345                 350
Lys Ser Glu Glu Glu Val Ala Ala Arg Arg Ala Arg Val Val Trp Cys
        355                 360                 365
Ala Val Gly Glu Gln Glu Leu Arg Lys Cys Asn Gln Trp Ser Gly Leu
    370                 375                 380
Ser Glu Gly Ser Val Thr Cys Ser Ser Ala Ser Thr Thr Glu Asp Cys
385                 390                 395                 400
Ile Ala Leu Val Leu Lys Gly Glu Ala Asp Ala Met Ser Leu Asp Gly
                405                 410                 415
Gly Tyr Val Tyr Thr Ala Cys Lys Cys Gly Leu Val Pro Val Leu Ala
            420                 425                 430
Glu Asn Tyr Lys Ser Gln Gln Ser Ser Asp Pro Asp Pro Asn Cys Val
        435                 440                 445
Asp Arg Pro Val Glu Gly Tyr Leu Ala Val Ala Val Val Arg Arg Ser
    450                 455                 460
Asp Thr Ser Leu Thr Trp Asn Ser Val Lys Gly Lys Lys Ser Cys His
465                 470                 475                 480
Thr Ala Val Asp Arg Thr Ala Gly Trp Asn Ile Pro Met Gly Leu Leu
                485                 490                 495

Phe Asn Gln Thr Gly Ser Cys Lys Phe Asp Glu Tyr Phe Ser Gln Ser
            500                 505                 510
Cys Ala Pro Gly Ser Asp Pro Arg Ser Asn Leu Cys Ala Leu Cys Ile
        515                 520                 525
Gly Asp Glu Gln Gly Glu Asn Lys Cys Val Pro Asn Ser Asn Glu Arg
    530                 535                 540
Tyr Tyr Gly Tyr Thr Gly Ala Phe Arg Cys Leu Ala Glu Asn Ala Gly
545                 550                 555                 560
Asp Val Ala Phe Val Lys Asp Val Thr Val Leu Gln Asn Thr Asp Gly
                565                 570                 575
Asn Asn Asn Glu Ala Trp Ala Lys Asp Leu Lys Leu Ala Asp Phe Ala
            580                 585                 590
Leu Leu Cys Leu Asp Gly Lys Arg Lys Pro Val Thr Glu Ala Arg Ser
        595                 600                 605
Cys His Leu Ala Met Ala Pro Asn His Ala Val Val Ser Arg Met Asp
    610                 615                 620
Lys Val Glu Arg Leu Lys Gln Val Leu Leu His Gln Gln Ala Lys Phe
625                 630                 635                 640
Gly Arg Asn Gly Ser Asp Cys Pro Asp Lys Phe Cys Leu Phe Gln Ser
                645                 650                 655
Glu Thr Lys Asn Leu Leu Phe Asn Asp Asn Thr Glu Cys Leu Ala Arg
            660                 665                 670
Leu His Gly Lys Thr Thr Tyr Glu Lys Tyr Leu Gly Pro Gln Tyr Val
        675                 680                 685
Ala Gly Ile Thr Asn Leu Lys Lys Cys Ser Thr Ser Pro Leu Leu Glu
    690                 695                 700

Ala Cys Glu Phe Leu Arg Lys
705                 710

<210> 10
<211> 21
<212> PRT
<213> Homo sapiens

<400> 10
Gly Ala Thr Met Ser Ala Leu Ile Leu Thr Ala Cys Ile Ile Trp Cys
1               5                   10                  15
Ile Cys Ser Ile Lys
            20

<210> 11
<211> 21
<212> PRT
<213> Homo sapiens

<400> 11
Asp Pro Arg Val Asn Pro Leu Lys Met Ser Pro His Ser Leu Thr Cys
1               5                   10                  15
Val Thr Ser Ser His
            20

<210> 12

<211> 63

<212> DNA

<213> Homo sapiens

<400> 12

gaccccaggg tcaatccgct gaagatgtct ccacactccc tgacctgcgt tacatcttcc 60

cac 63

<210> 13

<211> 63

<212> DNA

<213> Homo sapiens

<400> 13

gaccccaggg tcaatccgct gaagatgtct ccacactccc tgacctgcgt tacatcttcc 60

cac 63

<210> 14

<211> 1387

<212> PRT

<213> Anas platyrhynchos

<400> 14

Met Ala Gly Ala Ala Arg Gly Leu Leu Trp Ala Ala Leu Ser Leu Cys

1               5                   10                  15

Leu Leu Pro Glu Pro Leu Arg Ala Ala His Ile Lys Lys Ala Glu Ala

            20                  25                  30
Ala Ala Ala Gly Gly Gly Gly Gly Gly Val Gly Gly Glu Leu Arg Tyr
        35                  40                  45
Leu His Ala Ala Glu Leu Gly Gln Ala Leu Arg Asp Leu Val Ala Glu
    50                  55                  60
Ala Pro Pro Gly Leu Ala Arg Leu Phe Ser Ile Gly Arg Ser Val Glu
65                  70                  75                  80
Gly Arg Pro Leu Trp Val Leu Arg Leu Thr Ala Gly Leu Pro Glu Leu
                85                  90                  95
Pro Glu Ala Arg Gln Asp Gly Glu Lys Lys Lys Lys Glu Glu Glu Glu
            100                 105                 110
Glu Glu Glu Glu Glu Gly Glu Glu Gly Gly Gly Gly Ala Leu Pro Gly
        115                 120                 125
Arg Pro Gln Val Lys Leu Val Gly Asn Met His Gly Asp Glu Pro Leu
    130                 135                 140
Ala Arg Pro Leu Leu Leu Arg Leu Ala Gln Glu Leu Val Arg Gly Trp
145                 150                 155                 160
Ala Gly Gly Asp Glu Arg Leu Gly Arg Leu Leu Asn Thr Thr Asp Leu
                165                 170                 175
Tyr Leu Leu Pro Ser Leu Asn Pro Asp Gly Phe Glu Arg Ala Arg Glu
            180                 185                 190
Gly Asp Cys Gly Gly Gly Gly Gly Gly Glu Gly Gly Gly Glu Pro Gly
        195                 200                 205
Gly Arg Glu Asn Ser Arg Gly Arg Asp Leu Asn Arg Ser Phe Pro Asp
    210                 215                 220
Gln Phe Gly Ser Ala Gln Pro Asp Leu Glu Pro Val Pro Glu Val Arg

225                 230                 235                 240
Ala Leu Ile Ala Trp Met Arg Arg Asn Lys Phe Leu Leu Ser Gly Asn
                245                 250                 255
Leu His Gly Gly Ser Val Val Ala Ser Tyr Pro Tyr Asp Asp Ser Pro
            260                 265                 270
Thr His Arg Pro Thr Gly Val Tyr Ser Lys Ser Ala Asp Asp Glu Val
        275                 280                 285
Phe Lys Tyr Leu Ala Lys Ala Tyr Ala Ser His His Pro Ile Met Arg
    290                 295                 300
Thr Gly Lys Pro Asn Cys Pro Gly Glu Glu Gly Glu Thr Phe Gln Asp
305                 310                 315                 320
Gly Ile Thr Asn Gly Ala Gln Trp Tyr Asp Val Glu Gly Gly Met Gln
                325                 330                 335
Asp Tyr Asn Tyr Val Trp Ala Asn Cys Phe Glu Ile Thr Leu Glu Leu
            340                 345                 350
Ser Cys Cys Lys Tyr Pro Pro Thr Ser Glu Leu Gln Gln Glu Trp Glu
        355                 360                 365
Asn Asn Arg Glu Ser Leu Leu Thr Phe Ile Glu Lys Val His Ile Gly
    370                 375                 380
Val Lys Gly Tyr Val Arg Asp Ala Ile Thr Gly Ala Gly Leu Glu Asn
385                 390                 395                 400
Ala Thr Ile Val Val Ala Gly Ile Ala His Asn Ile Thr Ala Gly Lys
                405                 410                 415
Phe Gly Asp Tyr His Arg Leu Leu Val Pro Gly Thr Tyr Asn Val Thr
            420                 425                 430
Ala Val Val Met Gly Tyr Ala Pro Val Thr Lys Glu Asn Ile Glu Val

        435                 440                 445
Lys Glu Gly Asp Ala Thr Val Val Asp Phe Ser Leu Gln Pro Thr Val
    450                 455                 460
Val Ala Pro Asp Pro Asn Leu Thr Gln Phe Thr Ala Thr Pro Ala Pro
465                 470                 475                 480
Leu Ser Thr Leu Thr Pro Ser Val Ala Gln Ala Glu Pro Pro Ala Thr
                485                 490                 495
Thr Ser Leu His Arg Ala Val Gln Pro Val Asp Phe Arg His His His
            500                 505                 510
Phe Ser Asp Met Glu Ile Phe Leu Arg Arg Tyr Ala Asn Glu Tyr Pro
        515                 520                 525
Ser Ile Thr Arg Leu Tyr Ser Val Gly Lys Ser Val Glu Leu Arg Glu
    530                 535                 540
Leu Tyr Val Met Glu Ile Ser Asp Asn Pro Gly Val His Glu Ala Gly
545                 550                 555                 560
Glu Pro Glu Phe Lys Tyr Ile Gly Asn Met His Gly Asn Glu Val Val
                565                 570                 575
Gly Arg Glu Leu Leu Leu Asn Leu Ile Glu Tyr Leu Cys Lys Asn Phe
            580                 585                 590
Gly Thr Asp Pro Glu Val Thr Asp Leu Val Gln Ser Thr Arg Ile His
        595                 600                 605
Ile Met Pro Ser Met Asn Pro Asp Gly Tyr Glu Lys Ser Gln Glu Gly
    610                 615                 620
Asp Arg Gly Gly Thr Val Gly Arg Asn Asn Ser Asn Asn Tyr Asp Leu
625                 630                 635                 640
Asn Arg Asn Phe Pro Asp Gln Phe Phe Gln Val Thr Asp Pro Pro Gln

                645                 650                 655
Pro Glu Thr Leu Ala Val Met Ser Trp Leu Lys Thr Tyr Pro Phe Val
            660                 665                 670
Leu Ser Ala Asn Leu His Gly Gly Ser Leu Val Val Asn Tyr Pro Phe
        675                 680                 685
Asp Asp Asp Glu Gln Gly Ile Ala Ile Tyr Ser Lys Ser Pro Asp Asp
    690                 695                 700
Ala Val Phe Gln Gln Leu Ala Leu Ser Tyr Ser Lys Glu Asn Lys Lys
705                 710                 715                 720
Met Tyr Gln Gly Ser Pro Cys Lys Asp Leu Tyr Pro Thr Glu Tyr Phe
                725                 730                 735
Pro His Gly Ile Thr Asn Gly Ala Gln Trp Tyr Asn Val Pro Gly Gly
            740                 745                 750
Met Gln Asp Trp Asn Tyr Leu Asn Thr Asn Cys Phe Glu Val Thr Ile
        755                 760                 765
Glu Leu Gly Cys Val Lys Tyr Pro Lys Ala Glu Glu Leu Pro Lys Tyr
    770                 775                 780
Trp Glu Gln Asn Arg Arg Ser Leu Leu Gln Phe Ile Lys Gln Val His
785                 790                 795                 800
Arg Gly Ile Trp Gly Phe Val Leu Asp Ala Thr Asp Gly Arg Gly Ile
                805                 810                 815
Leu Asn Ala Thr Ile Ser Val Ala Asp Ile Asn His Pro Val Thr Thr
            820                 825                 830
Tyr Lys Asp Gly Asp Tyr Trp Arg Leu Leu Val Gln Gly Thr Tyr Lys
        835                 840                 845
Val Thr Ala Ser Ala Arg Gly Tyr Asp Pro Val Thr Lys Thr Val Glu

850                 855                 860

Val Asp Ser Lys Gly Gly Val Gln Val Asn Phe Thr Leu Ser Arg Thr

865                 870                 875                 880

Asp Ala Lys Val Glu Glu Gly Lys Val Pro Val Leu Asn Thr Pro Asp

                885                 890                 895

Thr Ser Asp Pro Asn Glu Lys Glu Phe Glu Thr Leu Ile Lys Asp Leu

            900                 905                 910

Ser Ala Glu Asn Gly Leu Glu Arg Leu Leu Leu Ala Ser Ser Gly Lys

        915                 920                 925

Val Ser Pro Tyr Arg Tyr Arg Pro Tyr Lys Asp Leu Ser Glu Phe Leu

    930                 935                 940

Arg Gly Leu Tyr Leu Asn Tyr Pro His Ile Thr Asn Leu Thr Ser Leu

945                 950                 955                 960

Gly Gln Ser Val Glu Phe Arg Gln Ile Trp Ser Leu Glu Ile Ser Asn

                965                 970                 975

Lys Pro Asn His Ser Glu Pro Glu Glu Pro Lys Ile Arg Phe Val Ala

            980                 985                 990

Gly Ile His Gly Asn Ala Pro Val Gly Thr Glu Leu Leu Leu Ala Leu

        995                 1000                1005

Ala Glu Phe Leu Cys Met Asn Tyr Lys Lys Asn Ser Ala Val Thr Lys

    1010                1015                1020

Leu Ile Asp Arg Thr Arg Ile Val Ile Val Pro Ser Leu Asn Pro Asp

1025                1030                1035                1040

Gly Arg Glu Ile Ala Gln Glu Arg Gly Cys Thr Ser Lys Leu Gly His

                1045                1050                1055

Ala Asn Ala His Gly Arg Asp Leu Asp Thr Asp Phe Thr Ser Asn Tyr

1060                1065                1070
Ser Arg Tyr Ser Gly Thr Arg Glu Pro Glu Thr Lys Ala Ile Ile Glu
        1075                1080                1085
Asn Leu Ile Leu Lys Gln Asp Phe Ser Leu Ser Val Ala Leu Asp Gly
    1090                1095                1100
Gly Ser Leu Leu Val Thr Tyr Pro Phe Asp Lys Pro Ala Gln Thr Val
1105                1110                1115                1120
Glu Asn Lys Asp Thr Leu Lys His Leu Ala Ser Val Tyr Ala Asn Asn
                1125                1130                1135
His Pro Leu Met His Leu Gly Gln Pro Gly Cys Pro Asn Lys Ser Asp
            1140                1145                1150
Glu Asn Ile Pro Gly Gly Val Ile Arg Gly Ser Glu Trp His Ser His
        1155                1160                1165
Leu Gly Ser Met Lys Asp Phe Ser Val Thr Phe Gly His Cys Pro Glu
    1170                1175                1180
Ile Thr Val Tyr Thr Ser Cys Cys Tyr Phe Pro Ser Ala Gly Gln Leu
1185                1190                1195                1200
Pro Gly Leu Trp Ala Asp His Arg Lys Ser Leu Leu Ser Met Leu Val
                1205                1210                1215
Glu Val His Lys Gly Val His Gly Phe Val Gln Asp Lys Ser Gly Lys
            1220                1225                1230
Ala Ile Ser Lys Ala Thr Ile Val Leu Asn Glu Gly Leu Arg Val Tyr
        1235                1240                1245
Thr Lys Glu Gly Gly Tyr Phe His Val Leu Leu Ala Pro Gly Leu His
    1250                1255                1260
Asn Ile Asn Ala Ile Ala Asp Gly Tyr Gln Gln Lys His Met Lys Val

1265                1270                1275                1280

Leu Val Arg His Asp Ala Pro Ser Ser Val Phe Ile Val Phe Asp Met

                1285                1290                1295

Glu Asn Arg Ile Phe Gly Leu Pro Arg Glu Leu Val Val Thr Val Ala

            1300                1305                1310

Gly Ala Ser Met Ser Ala Leu Val Leu Thr Ala Cys Ile Ile Trp Cys

        1315                1320                1325

Val Cys Ser Ile Lys Ser Asn Arg His Lys Asp Gly Phe Pro Thr Leu

    1330                1335                1340

Arg Gln His His Asp Asp Tyr Glu Asp Glu Ile Arg Met Met Ser Thr

1345                1350                1355                1360

Gly Ser Lys Lys Ser Leu Leu Ser His Glu Phe Gln Asp Glu Thr Asp

                1365                1370                1375

Thr Glu Glu Glu Thr Leu Tyr Ser Ser Lys His

            1380                1385

<210> 15

<211> 1024

<212> PRT

<213> Anas platyrhynchos

<400> 15

Met Arg Leu Ser Leu Phe Ile Ser Asp Ala Ala Arg Gly Gly Pro Arg

 1               5                  10                  15

His Leu Arg Pro Ala Gly Gly Gly Gly Gln His Arg Gln Gln Gln His

            20                  25                  30

Asn Gln Gln Gln Gln Arg Trp Val Gly Gly Gly Gly Gly Gly Gly Gly
        35                  40                  45
Gly Gly Gly Glu Ala Ala Arg Cys Ile Glu Gln Leu Leu Pro Arg His
    50                  55                  60
Asp Asp Phe Ser Arg Arg His Ile Gly Pro Arg Glu Gly Glu Lys Arg
65                  70                  75                  80
Glu Met Leu Arg Ala Leu Gly Val Gln Ser Val Glu Glu Leu Met Asp
                85                  90                  95
Lys Ala Ile Pro Gly Ser Ile Arg Leu Arg Arg Pro Leu Arg Met Glu
            100                 105                 110
Asp Pro Val Gly Glu Asn Glu Ile Leu Glu Thr Leu Tyr Asn Ile Ala
        115                 120                 125
Ser Lys Asn Lys Ile Trp Arg Ser Tyr Ile Gly Met Gly Tyr Tyr Asn
    130                 135                 140
Cys Ser Val Pro Gln Pro Ile Ala Arg Asn Leu Leu Glu Asn Ala Gly
145                 150                 155                 160
Trp Val Thr Gln Tyr Thr Pro Tyr Gln Pro Glu Val Ser Gln Gly Arg
                165                 170                 175
Leu Glu Ser Leu Leu Asn Tyr Gln Thr Met Val Cys Asp Ile Thr Gly
            180                 185                 190
Met Asp Val Ala Asn Ala Ser Leu Leu Asp Glu Gly Thr Ala Ala Ala
        195                 200                 205
Glu Ala Met Gln Leu Cys His Arg His Asn Lys Arg Arg Lys Phe Tyr
    210                 215                 220
Val Asp Ser Arg Cys His Pro Gln Thr Ile Ala Val Val Gln Thr Arg
225                 230                 235                 240

Ala Asn Tyr Thr Gly Val Ile Thr Glu Leu Lys Leu Pro His Glu Met
                245                 250                 255
Asp Phe Ser Gly Lys Asp Val Ser Gly Val Leu Phe Gln Tyr Pro Asp
            260                 265                 270
Thr Glu Gly Lys Val Glu Asp Phe Ser Glu Leu Val Glu Arg Ala His
        275                 280                 285
Gln Asn Gly Thr Leu Ala Cys Cys Ala Thr Asp Leu Leu Ala Leu Cys
    290                 295                 300
Ile Leu Lys Pro Pro Gly Glu Phe Gly Val Asp Val Val Leu Gly Ser
305                 310                 315                 320
Ser Gln Arg Phe Gly Val Pro Leu Cys Tyr Gly Gly Pro His Ala Ala
                325                 330                 335
Phe Phe Ala Val Lys Glu Asn Leu Val Arg Met Met Pro Gly Arg Met
            340                 345                 350
Val Gly Val Thr Arg Asp Ala Asn Gly Lys Glu Val Tyr Arg Leu Ala
        355                 360                 365
Leu Gln Thr Arg Glu Gln His Ile Arg Arg Asp Lys Ala Thr Ser Asn
    370                 375                 380
Ile Cys Thr Ala Gln Ala Leu Leu Ala Asn Met Ala Ala Met Phe Gly
385                 390                 395                 400
Val Tyr His Gly Ser Asp Gly Leu Arg Asp Ile Ala Arg Arg Val His
                405                 410                 415
Asn Ala Thr Leu Ile Leu Ala Glu Gly Leu Arg Arg Ala Gly His Lys
            420                 425                 430
Leu His His Asp Leu Phe Phe Asp Thr Leu Thr Val Thr Cys Gly Cys
        435                 440                 445

Ser Val Lys Glu Val Leu Asp Arg Ala Ala Leu Arg Lys Ile Asn Phe
    450                 455                 460
Arg Ile Tyr Ser Asp Gly Arg Leu Gly Val Ser Leu Asp Glu Thr Val
465                 470                 475                 480
Ser Glu Lys Asp Leu Asp Asp Ile Leu Trp Ile Phe Gly Cys Glu Ser
                485                 490                 495
Ser Ala Glu Leu Ile Ala Glu Gly Met Gly Glu Glu Thr Lys Gly Ile
            500                 505                 510
Leu Ser Thr Pro Phe Lys Arg Thr Ser Lys Phe Leu Thr His Gln Val
        515                 520                 525
Phe Asn Ser Tyr His Ser Glu Thr Asn Ile Val Arg Tyr Met Lys Arg
    530                 535                 540
Leu Glu Asn Lys Asp Ile Ser Leu Val His Ser Met Ile Pro Leu Gly
545                 550                 555                 560
Ser Cys Thr Met Lys Leu Asn Ser Ser Ala Glu Leu Ala Pro Ile Ser
                565                 570                 575
Trp Lys Glu Phe Ala Asn Ile His Pro Phe Val Pro Leu Asp Gln Ala
            580                 585                 590
Gln Gly Tyr Gln Gln Leu Phe Lys Asp Leu Glu Lys Asp Leu Cys Glu
        595                 600                 605
Ile Thr Gly Tyr Asp Lys Ile Ser Phe Gln Pro Asn Ser Gly Ala Gln
    610                 615                 620
Gly Glu Tyr Ala Gly Leu Ala Ala Ile Lys Ala Tyr Leu Asn Ala Lys
625                 630                 635                 640
Gly Glu Arg His Arg Ser Val Cys Leu Ile Pro Arg Ser Ala His Gly
                645                 650                 655

Thr Asn Pro Ala Ser Ala Gln Met Ala Gly Met Lys Ile Gln Pro Val
            660                 665                 670
Glu Val Asp Lys Asn Gly Ser Ile Asp Ile Ser His Leu Lys Ala Met
        675                 680                 685
Val Asp Lys His Lys Glu Asn Leu Ala Ala Ile Met Ile Thr Tyr Pro
    690                 695                 700
Ser Thr Asn Gly Val Phe Glu Glu Glu Ile Gly Asp Val Cys Glu Leu
705                 710                 715                 720
Ile His Lys Asn Gly Gly Gln Val Tyr Leu Asp Gly Ala Asn Met Asn
                725                 730                 735
Ala Gln Val Gly Leu Cys Arg Pro Gly Asp Tyr Gly Ser Asp Val Ser
            740                 745                 750
His Leu Asn Leu His Lys Thr Phe Cys Ile Pro His Gly Gly Gly Gly
        755                 760                 765
Pro Gly Met Gly Pro Ile Gly Val Lys Lys His Leu Ala Pro Tyr Leu
    770                 775                 780
Pro Thr His Pro Val Ile Lys Ile Gln Thr Asp Lys Asp Ala Cys Pro
785                 790                 795                 800
Leu Gly Thr Val Ser Ala Ala Pro Trp Gly Ser Ser Ala Ile Leu Pro
                805                 810                 815
Ile Ser Trp Val Tyr Ile Lys Thr Met Gly Ala Lys Gly Leu Lys His
            820                 825                 830
Ala Ser Glu Val Ala Ile Leu Asn Ala Asn Tyr Met Ala Lys Arg Leu
        835                 840                 845
Glu Lys His Tyr Lys Ile Leu Phe Arg Gly Val Arg Gly Tyr Val Ala
    850                 855                 860

His Glu Phe Ile Leu Asp Thr Arg Pro Phe Lys Lys Thr Ala Asn Ile
865                 870                 875                 880
Glu Ala Val Asp Leu Ala Lys Arg Leu Gln Asp Tyr Gly Phe His Ala
                885                 890                 895
Pro Thr Met Ser Trp Pro Val Ala Gly Thr Leu Met Ile Glu Pro Thr
            900                 905                 910
Glu Ser Glu Asp Lys Ala Glu Leu Asp Arg Phe Cys Asp Ala Met Ile
        915                 920                 925
Ser Ile Arg Gln Glu Ile Ala Glu Ile Glu Glu Gly Arg Met Asp Pro
    930                 935                 940
Gln Ile Asn Pro Leu Lys Met Ser Pro His Thr Leu Asn Cys Val Thr
945                 950                 955                 960
Ser Ser Lys Trp Asp Arg Pro Tyr Ser Arg Glu Val Ala Ala Phe Pro
                965                 970                 975
Leu Pro Phe Val Lys Pro Glu Ser Lys Phe Trp Pro Thr Ile Ala Arg
            980                 985                 990
Ile Asp Asp Ile Tyr Gly Asp Gln His Leu Val Cys Thr Cys Pro Pro
        995                 1000                1005
Met Glu Ala Tyr Glu Ser Pro Phe Ser Glu Gln Lys Arg Ala Ser Ser
    1010                1015                1020

<210> 16
<211> 1377
<212> PRT
<213> Homo sapiens

<400> 16

Met Ala Ser Gly Arg Asp Glu Arg Pro His Cys Val Gly Arg Leu Leu

1               5                   10                  15

Leu Leu Met Cys Leu Leu Leu Leu Gly Ser Ser Ala Arg Ala Ala His

            20                  25                  30

Ile Lys Lys Ala Glu Ala Thr Thr Thr Thr Thr Ser Ala Gly Ala Arg

        35                  40                  45

Gly Arg Gly Gln Phe Asp Arg Tyr Tyr His Glu Glu Glu Leu Glu Ser

    50                  55                  60

Ala Leu Arg Glu Ala Ala Ala Ala Gly Leu Pro Gly Leu Ala Arg Leu

65                  70                  75                  80

Phe Ser Ile Gly Arg Ser Val Glu Gly Arg Pro Leu Trp Val Leu Arg

                85                  90                  95

Leu Thr Ala Gly Leu Gly Ser Leu Ile Pro Glu Gly Asp Ala Gly Pro

            100                 105                 110

Asp Ala Ala Gly Pro Asp Ala Ala Gly Pro Leu Leu Pro Gly Arg Pro

        115                 120                 125

Gln Val Lys Leu Val Gly Asn Met His Gly Asp Glu Thr Val Ser Arg

    130                 135                 140

Gln Val Leu Ile Tyr Leu Ala Arg Glu Leu Ala Ala Leu Pro Pro Gly

145                 150                 155                 160

Asp Pro Arg Leu Val Arg Leu Leu Asn Thr Thr Asp Val Tyr Leu Leu

                165                 170                 175

Pro Ser Leu Asn Pro Asp Gly Phe Glu Arg Ala Arg Glu Gly Asp Cys

            180                 185                 190

Gly Phe Gly Asp Gly Gly Pro Ser Gly Ala Ser Gly Arg Asp Asn Ser

        195                 200                 205
Arg Gly Arg Asp Leu Asn Arg Ser Phe Pro Asp Gln Phe Ser Thr Gly
    210                 215                 220
Glu Pro Pro Ala Leu Asp Glu Val Pro Glu Val Arg Ala Leu Ile Glu
225                 230                 235                 240
Trp Ile Arg Arg Asn Lys Phe Val Leu Ser Gly Asn Leu His Gly Gly
                245                 250                 255
Ser Val Val Ala Ser Tyr Pro Phe Asp Asp Ser Pro Glu His Lys Ala
            260                 265                 270
Thr Gly Ile Tyr Ser Lys Thr Ser Asp Asp Glu Val Phe Lys Tyr Leu
        275                 280                 285
Ala Lys Ala Tyr Ala Ser Asn His Pro Ile Met Lys Thr Gly Glu Pro
    290                 295                 300
His Cys Pro Gly Asp Glu Asp Glu Thr Phe Lys Asp Gly Ile Thr Asn
305                 310                 315                 320
Gly Ala His Trp Tyr Asp Val Glu Gly Gly Met Gln Asp Tyr Asn Tyr
                325                 330                 335
Val Trp Ala Asn Cys Phe Glu Ile Thr Leu Glu Leu Ser Cys Cys Lys
            340                 345                 350
Tyr Pro Pro Ala Ser Gln Leu Arg Gln Glu Trp Glu Asn Asn Arg Glu
        355                 360                 365
Ser Leu Ile Thr Leu Ile Glu Lys Val His Ile Gly Val Lys Gly Phe
    370                 375                 380
Val Lys Asp Ser Ile Thr Gly Ser Gly Leu Glu Asn Ala Thr Ile Ser
385                 390                 395                 400
Val Ala Gly Ile Asn His Asn Ile Thr Thr Gly Arg Phe Gly Asp Phe

                405                 410                 415
Tyr Arg Leu Leu Val Pro Gly Thr Tyr Asn Leu Thr Val Val Leu Thr
            420                 425                 430
Gly Tyr Met Pro Leu Thr Val Thr Asn Val Val Val Lys Glu Gly Pro
        435                 440                 445
Ala Thr Glu Val Asp Phe Ser Leu Arg Pro Thr Val Thr Ser Val Ile
    450                 455                 460
Pro Asp Thr Thr Glu Ala Val Ser Thr Ala Ser Thr Val Ala Ile Pro
465                 470                 475                 480
Asn Ile Leu Ser Gly Thr Ser Ser Ser Cys Gln Pro Ile Gln Pro Lys
                485                 490                 495
Asp Phe His His His His Phe Pro Asp Met Glu Ile Phe Leu Arg Arg
            500                 505                 510
Phe Ala Asn Glu Tyr Pro Asn Ile Thr Arg Leu Tyr Ser Leu Gly Lys
        515                 520                 525
Ser Val Glu Ser Arg Glu Leu Tyr Val Met Glu Ile Ser Asp Asn Pro
    530                 535                 540
Gly Val His Glu Pro Gly Glu Pro Glu Phe Lys Tyr Ile Gly Asn Met
545                 550                 555                 560
His Gly Asn Glu Val Val Gly Arg Glu Leu Leu Leu Asn Leu Ile Glu
                565                 570                 575
Tyr Leu Cys Lys Asn Phe Gly Thr Asp Pro Glu Val Thr Asp Leu Val
            580                 585                 590
His Asn Thr Arg Ile His Leu Met Pro Ser Met Asn Pro Asp Gly Tyr
        595                 600                 605
Glu Lys Ser Gln Glu Gly Asp Ser Ile Ser Val Ile Gly Arg Asn Asn

    610                 615                 620
Ser Asn Asn Phe Asp Leu Asn Arg Asn Phe Pro Asp Gln Phe Val Gln
625                 630                 635                 640
Ile Thr Asp Pro Thr Gln Pro Glu Thr Ile Ala Val Met Ser Trp Met
                645                 650                 655
Lys Ser Tyr Pro Phe Val Leu Ser Ala Asn Leu His Gly Gly Ser Leu
            660                 665                 670
Val Val Asn Tyr Pro Phe Asp Asp Asp Glu Gln Gly Leu Ala Thr Tyr
        675                 680                 685
Ser Lys Ser Pro Asp Asp Ala Val Phe Gln Gln Ile Ala Leu Ser Tyr
    690                 695                 700
Ser Lys Glu Asn Ser Gln Met Phe Gln Gly Arg Pro Cys Lys Asn Met
705                 710                 715                 720
Tyr Pro Asn Glu Tyr Phe Pro His Gly Ile Thr Asn Gly Ala Ser Trp
                725                 730                 735
Tyr Asn Val Pro Gly Gly Met Gln Asp Trp Asn Tyr Leu Gln Thr Asn
            740                 745                 750
Cys Phe Glu Val Thr Ile Glu Leu Gly Cys Val Lys Tyr Pro Leu Glu
        755                 760                 765
Lys Glu Leu Pro Asn Phe Trp Glu Gln Asn Arg Arg Ser Leu Ile Gln
    770                 775                 780
Phe Met Lys Gln Val His Gln Gly Val Arg Gly Phe Val Leu Asp Ala
785                 790                 795                 800
Thr Asp Gly Arg Gly Ile Leu Asn Ala Thr Ile Ser Val Ala Glu Ile
                805                 810                 815
Asn His Pro Val Thr Thr Tyr Lys Thr Gly Asp Tyr Trp Arg Leu Leu

            820                 825                 830
Val Pro Gly Thr Tyr Lys Ile Thr Ala Ser Ala Arg Gly Tyr Asn Pro
        835                 840                 845
Val Thr Lys Asn Val Thr Val Lys Ser Glu Gly Ala Ile Gln Val Asn
    850                 855                 860
Phe Thr Leu Val Arg Ser Ser Thr Asp Ser Asn Asn Glu Ser Lys Lys
865                 870                 875                 880
Gly Lys Gly Ala Ser Ser Ser Thr Asn Asp Ala Ser Asp Pro Thr Thr
                885                 890                 895
Lys Glu Phe Glu Thr Leu Ile Lys Asp Leu Ser Ala Glu Asn Gly Leu
            900                 905                 910
Glu Ser Leu Met Leu Arg Ser Ser Ser Asn Leu Ala Leu Ala Leu Tyr
        915                 920                 925
Arg Tyr His Ser Tyr Lys Asp Leu Ser Glu Phe Leu Arg Gly Leu Val
    930                 935                 940
Met Asn Tyr Pro His Ile Thr Asn Leu Thr Asn Leu Gly Gln Ser Thr
945                 950                 955                 960
Glu Tyr Arg His Ile Trp Ser Leu Glu Ile Ser Asn Lys Pro Asn Val
                965                 970                 975
Ser Glu Pro Glu Glu Pro Lys Ile Arg Phe Val Ala Gly Ile His Gly
            980                 985                 990
Asn Ala Pro Val Gly Thr Glu Leu Leu Leu Ala Leu Ala Glu Phe Leu
        995                 1000                1005
Cys Leu Asn Tyr Lys Lys Asn Pro Ala Val Thr Gln Leu Val Asp Arg
    1010                1015                1020
Thr Arg Ile Val Ile Val Pro Ser Leu Asn Pro Asp Gly Arg Glu Arg

1025                1030                1035                1040
Ala Gln Glu Lys Asp Cys Thr Ser Lys Ile Gly Gln Thr Asn Ala Arg
                1045                1050                1055
Gly Lys Asp Leu Asp Thr Asp Phe Thr Asn Asn Ala Ser Gln Pro Glu
            1060                1065                1070
Thr Lys Ala Ile Ile Glu Asn Leu Ile Gln Lys Gln Asn Phe Ser Leu
        1075                1080                1085
Ser Val Ala Leu Asp Gly Gly Ser Met Leu Val Thr Tyr Pro Tyr Asp
    1090                1095                1100
Lys Pro Val Gln Thr Val Glu Asn Lys Glu Thr Leu Lys His Leu Ala
1105                1110                1115                1120
Ser Leu Tyr Ala Asn Asn His Pro Ser Met His Met Gly Gln Pro Ser
                1125                1130                1135
Cys Pro Asn Lys Ser Asp Glu Asn Ile Pro Gly Gly Val Met Arg Gly
            1140                1145                1150
Ala Glu Trp His Ser His Leu Gly Ser Met Lys Asp Tyr Ser Val Thr
        1155                1160                1165
Tyr Gly His Cys Pro Glu Ile Thr Val Tyr Thr Ser Cys Cys Tyr Phe
    1170                1175                1180
Pro Ser Ala Ala Arg Leu Pro Ser Leu Trp Ala Asp Asn Lys Arg Ser
1185                1190                1195                1200
Leu Leu Ser Met Leu Val Glu Val His Lys Gly Val His Gly Phe Val
                1205                1210                1215
Lys Asp Lys Thr Gly Lys Pro Ile Ser Lys Ala Val Ile Val Leu Asn
            1220                1225                1230
Glu Gly Ile Lys Val Gln Thr Lys Glu Gly Gly Tyr Phe His Val Leu

1235                1240                1245

Leu Ala Pro Gly Val His Asn Ile Ile Ala Ile Ala Asp Gly Tyr Gln

    1250                1255                1260

Gln Gln His Ser Gln Val Phe Val His His Asp Ala Ala Ser Ser Val

1265                1270                1275                1280

Val Ile Val Phe Asp Thr Asp Asn Arg Ile Phe Gly Leu Pro Arg Glu

                1285                1290                1295

Leu Val Val Thr Val Ser Gly Ala Thr Met Ser Ala Leu Ile Leu Thr

            1300                1305                1310

Ala Cys Ile Ile Trp Cys Ile Cys Ser Ile Lys Ser Asn Arg His Lys

        1315                1320                1325

Asp Gly Phe His Arg Leu Arg Gln His His Asp Glu Tyr Glu Asp Glu

    1330                1335                1340

Ile Arg Met Met Ser Thr Gly Ser Lys Lys Ser Leu Leu Ser His Glu

1345                1350                1355                1360

Phe Gln Asp Glu Thr Asp Thr Glu Glu Glu Thr Leu Tyr Ser Ser Lys

                1365                1370                1375

His

<210> 17

<211> 1020

<212> PRT

<213> Homo sapiens

<400> 17

Met Gln Ser Cys Ala Arg Ala Trp Gly Leu Arg Leu Gly Arg Gly Val

1                   5                   10                  15
Gly Gly Gly Arg Arg Leu Ala Gly Gly Ser Gly Pro Cys Trp Ala Pro
                20                  25                  30
Arg Ser Arg Asp Ser Ser Ser Gly Gly Gly Asp Ser Ala Ala Ala Gly
            35                  40                  45
Ala Ser Arg Leu Leu Glu Arg Leu Leu Pro Arg His Asp Asp Phe Ala
        50                  55                  60
Arg Arg His Ile Gly Pro Gly Asp Lys Asp Gln Arg Glu Met Leu Gln
65                  70                  75                  80
Thr Leu Gly Leu Ala Ser Ile Asp Glu Leu Ile Glu Lys Thr Val Pro
                85                  90                  95
Ala Asn Ile Arg Leu Lys Arg Pro Leu Lys Met Glu Asp Pro Val Cys
            100                 105                 110
Glu Asn Glu Ile Leu Ala Thr Leu His Ala Ile Ser Ser Lys Asn Gln
        115                 120                 125
Ile Trp Arg Ser Tyr Ile Gly Met Gly Tyr Tyr Asn Cys Ser Val Pro
    130                 135                 140
Gln Thr Ile Leu Arg Asn Leu Leu Glu Asn Ser Gly Trp Ile Thr Gln
145                 150                 155                 160
Tyr Thr Pro Tyr Gln Pro Glu Val Ser Gln Gly Arg Leu Glu Ser Leu
                165                 170                 175
Leu Asn Tyr Gln Thr Met Val Cys Asp Ile Thr Gly Leu Asp Met Ala
            180                 185                 190
Asn Ala Ser Leu Leu Asp Glu Gly Thr Ala Ala Ala Glu Ala Leu Gln
        195                 200                 205
Leu Cys Tyr Arg His Asn Lys Arg Arg Lys Phe Leu Val Asp Pro Arg

    210                 215                 220

Cys His Pro Gln Thr Ile Ala Val Val Gln Thr Arg Ala Lys Tyr Thr

225                 230                 235                 240

Gly Val Leu Thr Glu Leu Lys Leu Pro Cys Glu Met Asp Phe Ser Gly

                245                 250                 255

Lys Asp Val Ser Gly Val Leu Phe Gln Tyr Pro Asp Thr Glu Gly Lys

            260                 265                 270

Val Glu Asp Phe Thr Glu Leu Val Glu Arg Ala His Gln Ser Gly Ser

        275                 280                 285

Leu Ala Cys Cys Ala Thr Asp Leu Leu Ala Leu Cys Ile Leu Arg Pro

    290                 295                 300

Pro Gly Glu Phe Gly Val Asp Ile Ala Leu Gly Ser Ser Gln Arg Phe

305                 310                 315                 320

Gly Val Pro Leu Gly Tyr Gly Gly Pro His Ala Ala Phe Phe Ala Val

                325                 330                 335

Arg Glu Ser Leu Val Arg Met Met Pro Gly Arg Met Val Gly Val Thr

            340                 345                 350

Arg Asp Ala Thr Gly Lys Glu Val Tyr Arg Leu Ala Leu Gln Thr Arg

        355                 360                 365

Glu Gln His Ile Arg Arg Asp Lys Ala Thr Ser Asn Ile Cys Thr Ala

    370                 375                 380

Gln Ala Leu Leu Ala Asn Met Ala Ala Met Phe Arg Ile Tyr His Gly

385                 390                 395                 400

Ser His Gly Leu Glu His Ile Ala Arg Arg Val His Asn Ala Thr Leu

                405                 410                 415

Ile Leu Ser Glu Gly Leu Lys Arg Ala Gly His Gln Leu Gln His Asp

            420                 425                 430
Leu Phe Phe Asp Thr Leu Lys Ile His Cys Gly Cys Ser Val Lys Glu
        435                 440                 445
Val Leu Gly Arg Ala Ala Gln Arg Gln Ile Asn Phe Arg Leu Phe Glu
    450                 455                 460
Asp Gly Thr Leu Gly Ile Ser Leu Asp Glu Thr Val Asn Glu Lys Asp
465                 470                 475                 480
Leu Asp Asp Leu Leu Trp Ile Phe Gly Cys Glu Ser Ser Ala Glu Leu
                485                 490                 495
Val Ala Glu Ser Met Gly Glu Glu Cys Arg Gly Ile Pro Gly Ser Val
            500                 505                 510
Phe Lys Arg Thr Ser Pro Phe Leu Thr His Gln Val Phe Asn Ser Tyr
        515                 520                 525
His Ser Glu Thr Asn Ile Val Arg Tyr Met Lys Lys Leu Glu Asn Lys
    530                 535                 540
Asp Ile Ser Leu Val His Ser Met Ile Pro Leu Gly Ser Cys Thr Met
545                 550                 555                 560
Lys Leu Asn Ser Ser Ser Glu Leu Ala Pro Ile Thr Trp Lys Glu Phe
                565                 570                 575
Ala Asn Ile His Pro Phe Val Pro Leu Asp Gln Ala Gln Gly Tyr Gln
            580                 585                 590
Gln Leu Phe Arg Glu Leu Glu Lys Asp Leu Cys Glu Leu Thr Gly Tyr
        595                 600                 605
Asp Gln Val Cys Phe Gln Pro Asn Ser Gly Ala Gln Gly Glu Tyr Ala
    610                 615                 620
Gly Leu Ala Thr Ile Arg Ala Tyr Leu Asn Gln Lys Gly Glu Gly His

625                 630                 635                 640
Arg Thr Val Cys Leu Ile Pro Lys Ser Ala His Gly Thr Asn Pro Ala
                645                 650                 655
Ser Ala His Met Ala Gly Met Lys Ile Gln Pro Val Glu Val Asp Lys
            660                 665                 670
Tyr Gly Asn Ile Asp Ala Val His Leu Lys Ala Met Val Asp Lys His
        675                 680                 685
Lys Glu Asn Leu Ala Ala Ile Met Ile Thr Tyr Pro Ser Thr Asn Gly
    690                 695                 700
Val Phe Glu Glu Asn Ile Ser Asp Val Cys Asp Leu Ile His Gln His
705                 710                 715                 720
Gly Gly Gln Val Tyr Leu Asp Gly Ala Asn Met Asn Ala Gln Val Gly
                725                 730                 735
Ile Cys Arg Pro Gly Asp Phe Gly Ser Asp Val Ser His Leu Asn Leu
            740                 745                 750
His Lys Thr Phe Cys Ile Pro His Gly Gly Gly Gly Pro Gly Met Gly
        755                 760                 765
Pro Ile Gly Val Lys Lys His Leu Ala Pro Phe Leu Pro Asn His Pro
    770                 775                 780
Val Ile Ser Leu Lys Arg Asn Glu Asp Ala Cys Pro Val Gly Thr Val
785                 790                 795                 800
Ser Ala Ala Pro Trp Gly Ser Ser Ser Ile Leu Pro Ile Ser Trp Ala
                805                 810                 815
Tyr Ile Lys Met Met Gly Gly Lys Gly Leu Lys Gln Ala Thr Glu Thr
            820                 825                 830
Ala Ile Leu Asn Ala Asn Tyr Met Ala Lys Arg Leu Glu Thr His Tyr

        835                 840                 845
Arg Ile Leu Phe Arg Gly Ala Arg Gly Tyr Val Gly His Glu Phe Ile
    850                 855                 860
Leu Asp Thr Arg Pro Phe Lys Lys Ser Ala Asn Ile Glu Ala Val Asp
865                 870                 875                 880
Val Ala Lys Arg Leu Gln Asp Tyr Gly Phe His Ala Pro Thr Met Ser
                885                 890                 895
Trp Pro Val Ala Gly Thr Leu Met Val Glu Pro Thr Glu Ser Glu Asp
            900                 905                 910
Lys Ala Glu Leu Asp Arg Phe Cys Asp Ala Met Ile Ser Ile Arg Gln
        915                 920                 925
Glu Ile Ala Asp Ile Glu Glu Gly Arg Ile Asp Pro Arg Val Asn Pro
    930                 935                 940
Leu Lys Met Ser Pro His Ser Leu Thr Cys Val Thr Ser Ser His Trp
945                 950                 955                 960
Asp Arg Pro Tyr Ser Arg Glu Val Ala Ala Phe Pro Leu Pro Phe Met
                965                 970                 975
Lys Pro Glu Asn Lys Phe Trp Pro Thr Ile Ala Arg Ile Asp Asp Ile
            980                 985                 990
Tyr Gly Asp Gln His Leu Val Cys Thr Cys Pro Pro Met Glu Val Tyr
        995                 1000                1005
Glu Ser Pro Phe Ser Glu Gln Lys Arg Ala Ser Ser
    1010                1015                1020

<210> 18
<211> 46

<212> PRT
<213> Homo sapiens

<400> 18
Gln Gly Asp Ser Ser Pro Cys Ser Ala Phe Glu Phe His Cys Leu Ser
1               5                   10                  15
Gly Glu Cys Ile His Ser Ser Trp Arg Cys Asp Gly Gly Pro Asp Cys
            20                  25                  30
Lys Asp Lys Ser Asp Glu Glu Asn Cys Ala Val Ala Thr Cys
        35                  40                  45

<210> 19
<211> 44
<212> PRT
<213> Homo sapiens

<400> 19
Asp Gly Gly Pro Asp Cys Lys Asp Lys Ser Asp Glu Glu Asn Cys Ala
1               5                   10                  15
Val Ala Thr Cys Arg Pro Asp Glu Phe Gln Cys Ser Asp Gly Asn Cys
            20                  25                  30
Ile His Gly Ser Arg Gln Cys Asp Arg Glu Tyr Asp
        35                  40

<210> 20
<211> 138

<212> DNA

<213> Homo sapiens

<400> 20

CAA GGG GAC AGT AGC CCC TGC TCG GCC TTC GAG TTC CAC TGC CTA AGT 48

GGC GAG TGC ATC CAC TCC AGC TGG CGC TGT GAT GGT GGC CCC GAC TGC 96

AAG GAC AAA TCT GAC GAG GAA AAC TGC GCT GTG GCC ACC TGT 138

<210> 21

<211> 132

<212> DNA

<213> Homo sapiens

<400> 21

GAT GGT GGC CCC GAC TGC AAG GAC AAA TCT GAC GAG GAA AAC TGC GCT 48

GTG GCC ACC TGT CGC CCT GAC GAA TTC CAG TGC TCT GAT GGA AAC TGC 96

ATC CAT GGC AGC CGG CAG TGT GAC CGG GAA TAT GAC 132

<210> 22

<211> 839

<212> DNA

<213> Homo sapiens

<400> 22

Ala Val Gly Asp Arg Cys Glu Arg Asn Glu Phe Gln Cys Gln Asp Gly

1               5                   10                  15

Lys Cys Ile Ser Tyr Lys Trp Val Cys Asp Gly Ser Ala Glu Cys Gln
            20                  25                  30
Asp Gly Ser Asp Glu Ser Gln Glu Thr Cys Leu Ser Val Thr Cys Lys
        35                  40                  45
Ser Gly Asp Phe Ser Cys Gly Gly Arg Val Asn Arg Cys Ile Pro Gln
    50                  55                  60
Phe Trp Arg Cys Asp Gly Gln Val Asp Cys Asp Asn Gly Ser Asp Glu
65                  70                  75                  80
Gln Gly Cys Pro Pro Lys Thr Cys Ser Gln Asp Glu Phe Arg Cys His
                85                  90                  95
Asp Gly Lys Cys Ile Ser Arg Gln Phe Val Cys Asp Ser Asp Arg Asp
            100                 105                 110
Cys Leu Asp Gly Ser Asp Glu Ala Ser Cys Pro Val Leu Thr Cys Gly
        115                 120                 125
Pro Ala Ser Phe Gln Cys Asn Ser Ser Thr Cys Ile Pro Gln Leu Trp
    130                 135                 140
Ala Cys Asp Asn Asp Pro Asp Cys Glu Asp Gly Ser Asp Glu Trp Pro
145                 150                 155                 160
Gln Arg Cys Arg Gly Leu Tyr Val Phe Gln Gly Asp Ser Ser Pro Cys
                165                 170                 175
Ser Ala Phe Glu Phe His Cys Leu Ser Gly Glu Cys Ile His Ser Ser
            180                 185                 190
Trp Arg Cys Asp Gly Gly Pro Asp Cys Lys Asp Lys Ser Asp Glu Glu
        195                 200                 205
Asn Cys Ala Val Ala Thr Cys Arg Pro Asp Glu Phe Gln Cys Ser Asp
    210                 215                 220

Gly Asn Cys Ile His Gly Ser Arg Gln Cys Asp Arg Glu Tyr Asp Cys
225                 230                 235                 240
Lys Asp Met Ser Asp Glu Val Gly Cys Val Asn Val Thr Leu Cys Glu
                245                 250                 255
Gly Pro Asn Lys Phe Lys Cys His Ser Gly Glu Cys Ile Thr Leu Asp
            260                 265                 270
Lys Val Cys Asn Met Ala Arg Asp Cys Arg Asp Trp Ser Asp Glu Pro
        275                 280                 285
Ile Lys Glu Cys Gly Thr Asn Glu Cys Leu Asp Asn Asn Gly Gly Cys
    290                 295                 300
Ser His Val Cys Asn Asp Leu Lys Ile Gly Tyr Glu Cys Leu Cys Pro
305                 310                 315                 320
Asp Gly Phe Gln Leu Val Ala Gln Arg Arg Cys Glu Asp Ile Asp Glu
                325                 330                 335
Cys Gln Asp Pro Asp Thr Cys Ser Gln Leu Cys Val Asn Leu Glu Gly
            340                 345                 350
Gly Tyr Lys Cys Gln Cys Glu Glu Gly Phe Gln Leu Asp Pro His Thr
        355                 360                 365
Lys Ala Cys Lys Ala Val Gly Ser Ile Ala Tyr Leu Phe Phe Thr Asn
    370                 375                 380
Arg His Glu Val Arg Lys Met Thr Leu Asp Arg Ser Glu Tyr Thr Ser
385                 390                 395                 400
Leu Ile Pro Asn Leu Arg Asn Val Val Ala Leu Asp Thr Glu Val Ala
                405                 410                 415
Ser Asn Arg Ile Tyr Trp Ser Asp Leu Ser Gln Arg Met Ile Cys Ser
            420                 425                 430

Thr Gln Leu Asp Arg Ala His Gly Val Ser Ser Tyr Asp Thr Val Ile
        435                 440                 445
Ser Arg Asp Ile Gln Ala Pro Asp Gly Leu Ala Val Asp Trp Ile His
    450                 455                 460
Ser Asn Ile Tyr Trp Thr Asp Ser Val Leu Gly Thr Val Ser Val Ala
465                 470                 475                 480
Asp Thr Lys Gly Val Lys Arg Lys Thr Leu Phe Arg Glu Asn Gly Ser
                485                 490                 495
Lys Pro Arg Ala Ile Val Val Asp Pro Val His Gly Phe Met Tyr Trp
            500                 505                 510
Thr Asp Trp Gly Thr Pro Ala Lys Ile Lys Lys Gly Gly Leu Asn Gly
        515                 520                 525
Val Asp Ile Tyr Ser Leu Val Thr Glu Asn Ile Gln Trp Pro Asn Gly
    530                 535                 540
Ile Thr Leu Asp Leu Leu Ser Gly Arg Leu Tyr Trp Val Asp Ser Lys
545                 550                 555                 560
Leu His Ser Ile Ser Ser Ile Asp Val Asn Gly Gly Asn Arg Lys Thr
                565                 570                 575
Ile Leu Glu Asp Glu Lys Arg Leu Ala His Pro Phe Ser Leu Ala Val
            580                 585                 590
Phe Glu Asp Lys Val Phe Trp Thr Asp Ile Ile Asn Glu Ala Ile Phe
        595                 600                 605
Ser Ala Asn Arg Leu Thr Gly Ser Asp Val Asn Leu Leu Ala Glu Asn
    610                 615                 620
Leu Leu Ser Pro Glu Asp Met Val Leu Phe His Asn Leu Thr Gln Pro
625                 630                 635                 640

Arg Gly Val Asn Trp Cys Glu Arg Thr Thr Leu Ser Asn Gly Gly Cys
                645                 650                 655
Gln Tyr Leu Cys Leu Pro Ala Pro Gln Ile Asn Pro His Ser Pro Lys
            660                 665                 670
Phe Thr Cys Ala Cys Pro Asp Gly Met Leu Leu Ala Arg Asp Met Arg
        675                 680                 685
Ser Cys Leu Thr Glu Ala Glu Ala Ala Val Ala Thr Gln Glu Thr Ser
    690                 695                 700
Thr Val Arg Leu Lys Val Ser Ser Thr Ala Val Arg Thr Gln His Thr
705                 710                 715                 720
Thr Thr Arg Pro Val Pro Asp Thr Ser Arg Leu Pro Gly Ala Thr Pro
                725                 730                 735
Gly Leu Thr Thr Val Glu Ile Val Thr Met Ser His Gln Ala Leu Gly
            740                 745                 750
Asp Val Ala Gly Arg Gly Asn Glu Lys Lys Pro Ser Ser Val Arg Ala
        755                 760                 765
Leu Ser Ile Val Leu Pro Ile Val Leu Leu Val Phe Leu Cys Leu Gly
    770                 775                 780
Val Phe Leu Leu Trp Lys Asn Trp Arg Leu Lys Asn Ile Asn Ser Ile
785                 790                 795                 800
Asn Phe Asp Asn Pro Val Tyr Gln Lys Thr Thr Glu Asp Glu Val His
                805                 810                 815
Ile Cys His Asn Gln Asp Gly Tyr Ser Tyr Pro Ser Arg Gln Met Val
            820                 825                 830
Ser Leu Glu Asp Asp Val Ala
        835

<210> 23

<211> 53

<212> PRT

<213> Artificial Sequence

<220>

<223> Description of Artificial Sequence: Synthesized peptide for peptid

e TAPA148 which is derived from human CD81

<400> 23

Thr Phe His Glu Thr Leu Asp Cys Cys Gly Ser Ser Thr Leu Thr Ala

1               5                   10                  15

Leu Thr Thr Ser Val Leu Lys Asn Asn Leu Cys Pro Ser Gly Ser Asn

            20                  25                  30

Ile Ile Ser Asn Leu Phe Lys Thr Asp Cys His Gln Lys Ile Asp Asp

        35                  40                  45

Leu Thr Ser Gly Lys

    50

<210> 24

<211> 36

<212> PRT

<213> Artificial Sequence

<220>

<223> Description of Artificial Sequence: Synthesized peptide for peptid
e bLF443 which is derived from bivine lactoferrin.

<400> 24
Leu Asp Cys Val Leu Arg Pro Thr Glu Gly Tyr Leu Ala Val Ala Val
1               5                   10                  15
Val Lys Lys Ala Asn Glu Gly Leu Thr Trp Asn Ser Leu Lys Asp Lys
            20                  25                  30
Lys Ser Cys His
        35

<210> 25
<211> 36
<212> PRT
<213> Artificial Sequence

<220>
<223> Description of Artificial Sequence: Syntehsized peptide for peptid
e hLF445 which is derived from human lactoferrin.

<400> 25
Pro Asn Cys Val Asp Arg Pro Val Glu Gly Tyr Leu Ala Val Ala Val
1               5                   10                  15
Val Arg Arg Ser Asp Thr Ser Leu Thr Trp Asn Ser Val Lys Gly Lys
            20                  25                  30
Lys Ser Cys His

        35

<210> 26

<211> 39

<212> PRT

<213> Artificial Sequence

<220>

<223> Description of Artificial Sequence: Synthesized peptide for peptid
e hLDLR194 which is derived from human LDL receptor.

<400> 26

Ser Ser Pro Cys Ser Ala Phe Glu Phe His Cys Leu Ser Gly Glu Cys
1               5                   10                  15

Ile His Ser Ser Trp Arg Cys Asp Gly Gly Pro Asp Cys Lys Asp Lys
            20                  25                  30

Ser Asp Glu Glu Asn Cys Ala
        35

<210> 27

<211> 37

<212> PRT

<213> Artificial Sequence

<220>

<223> Description of Artificial Sequence: Synthesized peptide for peptid

e hLDLR220 which is derived from human LDL receptor.

<400> 27
Pro Asp Cys Lys Asp Lys Ser Asp Glu Glu Asn Cys Ala Val Ala Thr
1               5                   10                  15
Cys Arg Pro Asp Glu Phe Gln Cys Ser Asp Gly Asn Cys Ile His Gly
            20                  25                  30
Ser Arg Gln Cys Asp
        35

<210> 28
<211> 33
<212> PRT
<213> Artificial Sequence

<220>
<223> Description of Artificial Sequence: Synthesized peptide for cyclic
 peptide cbLF445 which is derived from bovine lactoferrin.

<400> 28
Cys Val Leu Arg Pro Thr Glu Gly Tyr Leu Ala Val Ala Val Val Lys
1               5                   10                  15
Lys Ala Asn Glu Gly Leu Thr Trp Asn Ser Leu Lys Asp Lys Lys Ser
            20                  25                  30
Cys

<210> 29
<211> 29
<212> DNA
<213> Artificial Sequence

<220>
<223> Description of Artificial Sequence: Synthesized oligonucleotide fo
r interferon-alpha2b specific primer U-2.

<400> 29
ataggatccg cagcatctgc aacatctac                                    29

<210> 30
<211> 34
<212> DNA
<213> Artificial Sequence

<220>
<223> Description of Artificial Sequence: Synthesized oligonucleotide fo
r interferon-alpha2b specific primer L2.

<400> 30
tacaagcttg aaaatcattt ccatgttgaa ccag                              34

<210> 31
<211> 27

<212> DNA
<213> Artificial Sequence

<220>
<223> Description of Artificial Sequence: Synthesized oligonucleotide fo
r H3TER primer

<400> 31
cttaagcttt cttgcaagtt tgttgac                                          27

<210> 32
<211> 163
<212> DNA
<213> Artificial Sequence

<220>
<223> Description of Artificial Sequence: Synthesized nucleotide having
a sequence corresponding to the deduced HCV E2 binding region in CD81.

<400> 32
aagcttaaga agtaaggaac acgagacgct tgactgctgt ggctccagca cactgactgc 60
tttgaccacc tcagtgctca agaacaattt gtgtccctcg ggcagcaaca tcatcagcaa 120
cctcttcaag gaggactgcc accagaagat cgattgactt aag                    163

<210> 33
<211> 157

<212> DNA
<213> Artificial Sequence

<220>
<223> Description of Artificial Sequence: Synthesized nucleotide having
a sequence corresponding to the deduced HCV E2 binding region in bovine
lactoferrin.

<400> 33
aagcttaaga agtaaggaac acagtagcct agattgtgtg ctgagaccaa cggaagggta 60
ccttgccgtg gcagttgtca agaaagcaaa tgaggggctc acatggaatt ctctgaaaga 120
caagaagtcg tgccacaccg ccgtggactg acttaag 157

<210> 34
<211> 157
<212> DNA
<213> Artificial Sequence

<220>
<223> Description of Artificial Sequence: Synthesized nucleotide having
a sequence corresponding to the deduced HCV E2 binding region in human l
actoferrin.

<400> 34
aagcttaaga agtaaggaag accctgatcc taactgtgtg gatagacctg tggaaggata 60
tcttgctgtg gcggtggtta ggagatcaga cactagcctt acctggaact ctgtgaaagg 120

caagaagtcc tgccacaccg ccgtggactg acttaag 157

## Claims

1. A prophylactic or therapeutic agent for chronic hepatitis, comprising an oligopeptide having the following characteristics:
(A) having a binding affinity to a viral antigen protein;
(B) inhibiting binding of virus to a receptor protein on a target cell for said virus; and
(C) having homologies with said receptor protein at the amino acid sequence level.

2. The prophylactic or therapeutic agent for chronic hepatitis according to claim 1, wherein said oligopeptide comprises amino acid sequences essential to binding of a receptor protein to virus.

3. The prophylactic or therapeutic agent for chronic hepatitis according to claim 1 or 2, wherein said oligopeptide has 20% or more homology with said receptor protein at the amino acid sequence level.

4. A prophylactic or therapeutic agent for chronic type-C hepatitis, comprising a compound having the following characteristics:
(a) having a binding affinity to an envelope protein of HCV; and
(b) inhibiting binding of HCV to a receptor protein on a target cell for said HCV.

5. The prophylactic or therapeutic agent for chronic type-C hepatitis according to claim 4, wherein said compound is an oligopeptide having at least one kind of an amino acid sequence selected from the group consisting of SEQ ID NOs: 1,2 and 3, or a part thereof.

6. The prophylactic or therapeutic agent for chronic type-C hepatitis according to claim 4, wherein said compound is an oligopeptide having at least one kind of an amino acid sequence selected from the group consisting of SEQ ID NOs: 18 and 19, or a part thereof.

7. The prophylactic or therapeutic agent for chronic type-C hepatitis according to claim 4, wherein said compound is an oligopeptide or a part thereof, wherein said oligopeptide has the following characteristics:
I) consisting of 31 to 33 amino acids which are flanked by cysteine residues;
II) forming loop structure: and
III) comprising basic amino acid residues at a middle portion and C-terminal side of loop structure.

8. The prophylactic or therapeutic agent for chronic type-C hepatitis according to claim 4, wherein said compound is a cyclic oligopeptide comprising an oligopeptide or a part thereof, wherein said oligopeptide has the following characteristics:
I) consisting of 31 to 33 amino acids which are flanked by cysteine residues;
II) forming loop structure: and
III) comprising basic amino acid residues at a middle portion and C-terminal side of loop structure.

9. A prophylactic or therapeutic agent for chronic type-C hepatitis, comprising a nucleic acid having at least one kind of a nucleotide sequence selected from the group consisting of SEQ ID NOs: 4, 5 and 6.

10. A prophylactic or therapeutic agent for chronic type-C hepatitis, comprising a nucleic acid having at least one kind of a nucleotide sequence selected from the group consisting of SEQ ID NOs: 20 and 21.

11. A prophylactic or therapeutic agent for chronic type-B hepatitis, comprising a compound having the following characteristics:
(a') having a binding affinity to S antigen protein of HBV; and
(b') inhibiting binding of HBV to a receptor protein on a target cell for said HBV.

12. The prophylactic or therapeutic agent for chronic type-B hepatitis according to claim 7, comprising an oligopeptide having an amino acid sequence of SEQ ID NO: 10 or 11, or a part thereof.

13. The prophylactic or therapeutic agent for chronic type-B hepatitis, comprising a nucleic acid having a nucleotide sequence of SEQ ID NO: 12 or 13.

14. A fusion interferon containing an amino acid sequence of an oligopeptide having the following characteristics:
(A) having a binding affinity to a viral antigen protein;
(B) inhibiting binding of virus to a receptor protein on a target cell for said virus; and
(C) having homologies with said receptor protein at the amino acid sequence level.

15. The fusion interferon containing at least one kind of an amino acid sequence selected from the group consisting of SEQ ID NOs: 1, 2 and 3, or a part thereof.

16. The fusion interferon containing at least one kind of an amino acid sequence selected from the group consisting of SEQ ID NOs: 18 and 19, or a part thereof.

17. The fusion interferon containing an amino acid sequence of SEQ ID NO: 10 or 11, or a part thereof.

18. A method for treating chronic hepatitis in combination with the use of, **characterized by** using an oligopeptide or a part thereof in combination with interferon, wherein said oligopeptide has the following characteristics:
(A) having a binding affinity to a viral antigen protein;
(B) inhibiting binding of virus to a receptor protein on a target cell for said virus; and
(C) having homologies with said receptor protein at the amino acid sequence level.
